(19) Europäisches Patentamt
European Patent Office
Office européen des brevets

(11) EP 3 590 513 A1

(12) **EUROPEAN PATENT APPLICATION**
published in accordance with Art. 153(4) EPC

(43) Date of publication:
**08.01.2020 Bulletin 2020/02**

(21) Application number: **18761026.6**

(22) Date of filing: **28.02.2018**

(51) Int Cl.:
***A61K 31/472*** *(2006.01)*
***A61J 1/05*** *(2006.01)*
***A61K 9/08*** *(2006.01)*
***A61K 31/4725*** *(2006.01)*
***A61K 47/04*** *(2006.01)*
***A61K 47/10*** *(2017.01)*
***A61P 27/02*** *(2006.01)*
***A61P 27/06*** *(2006.01)*
***B65D 1/00*** *(2006.01)*
***B65D 65/20*** *(2006.01)*

(86) International application number:
**PCT/JP2018/007582**

(87) International publication number:
**WO 2018/159701 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **28.02.2017 JP 2017037500**

(71) Applicant: **Kowa Company, Ltd.**
**Nagoya-shi, Aichi 460-8625 (JP)**

(72) Inventor: **YAMAKITA, Yoshihiro**
**Fuji-shi**
**Shizuoka 417-8650 (JP)**

(74) Representative: **Schiener, Jens**
**Wächtershäuser & Hartz**
**Patentanwaltspartnerschaft mbB**
**Weinstraße 8**
**80333 München (DE)**

(54) **MEDICAL DRUG**

(57) To establish a technique for stably formulating an isoquinolin-6-amino derivative as an ophthalmic agent or the like. A pharmaceutical preparation is provided including an aqueous composition containing a compound represented by the following formula (1)

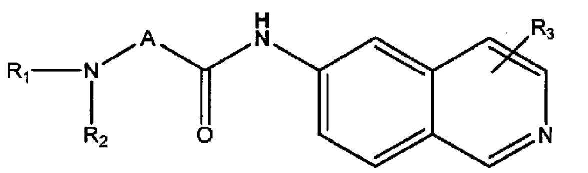

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and the formula (1) also includes a tautomer thereof) or a salt thereof or a solvate thereof, the aqueous composition being housed in a container made of a polyolefin resin.

EP 3 590 513 A1

## Description

Technical Field

The present invention relates to a pharmaceutical preparation and the like.

Background Art

Heretofore, several isoquinolin-6-amino derivatives have been known to be useful in prevention and treatment of eye diseases such as ocular hypertension and glaucoma. Specifically, for example, a compound represented by the following structural formula:

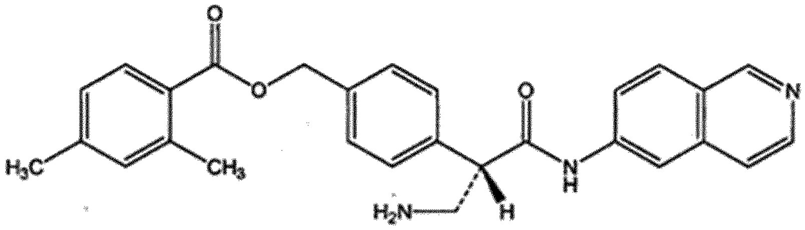

(Chemical Name: [4-[(2S)-3-amino-1-(isoquinolin-6-ylamino)-1-oxopropan-2-yl]phenyl]methyl 2,4-dimethylbenzoate), International Nonproprietary Name: netarsudil, hereinafter sometimes referred to as "netarsudil"), also known as AR-13324, has been reported to have pharmacological effects such as Rho kinase inhibitory effect and norepinephrine transporter inhibitory effect, and be useful in prevention and treatment of eye diseases such as ocular hypertension and glaucoma (for example, Patent Literature 1, and Non Patent Literatures 1 and 2).

Similarly, a compound represented by the following structural formula:

*rac*

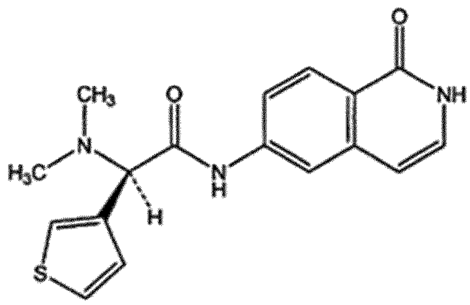

(Chemical Name: rac-(2R)-2-(dimethylamino)-N-(1-oxo-1,2-dihydroisoquinolin-6-yl)-2-(thiophen-3-yl)acetamide), International Nonproprietary Name: verosudil, hereinafter sometimes referred to as "verosudil"), also known as AR-12286, has been reported to have Rho kinase inhibitory effect and be useful in prevention and treatment of eye diseases such as ocular hypertension (for example, Patent Literature 2, and Non Patent Literatures 1 and 3).

Accordingly, it is very useful to establish a technique for stably formulating these isoquinolin-6-amino derivatives, for example, as an ophthalmic agent or the like.

Meanwhile, Patent Literature 3 states that a composition containing netarsudil or verosudil was prepared in a 150 mL plastic container. However, in the literature, each composition was prepared to use in pharmacological studies. The literature does not describe the stability of netarsudil and verosudil at all, neither describes the material and characteristics of the container at all.

Citation List

Patent Literature

Patent Literature 1: JP-A-2012-525386
Patent Literature 2: WO2009/091898

Patent Literature 3: JP-A-2016-515520

Non Patent Literature

Non Patent Literature 1: Bacharach J. et al., Ophthalmology 122(2) 302-7 (2015)
Non Patent Literature 2: Sturdivant JM. et al., Bioorg Med Chem Lett. 26(10) 2475-80 (2016)
Non Patent Literature 3: Williams RD. et al., Am. J. Ophthalmol. 152(5) 834-41 (2011)

Summary of Invention

Technical Problem

An object of the present invention is to establish a technique for stably formulating an isoquinolin-6-amino derivative such as netarsudil or verosudil as an ophthalmic agent or the like.

Solution to Problem

An ophthalmic agent or the like is usually a composition containing water (an aqueous composition). Thus, the present inventor prepared an aqueous composition comprising an isoquinolin-6-amino derivative such as netarsudil and housed the prepared composition in a container to check its storage property. However, unexpectedly, it was revealed that the content of isoquinolin-6-amino derivative is reduced with time due to exposure to light.

Therefore, the present inventor conducted further extensive studies to improve light stability of the isoquinolin-6-amino derivative in an aqueous composition. Consequently, the inventor has found that the content reduction due to exposure to light is suppressed by housing the aqueous composition comprising the isoquinolin-6-amino derivative in a specific container, i.e. a container formed of a polyolefin resin and that a pharmaceutical preparation having an improved light stability can be obtained, and have completed the present invention.

The present invention provides a pharmaceutical preparation comprising: an aqueous composition comprising a compound represented by the following formula (1)

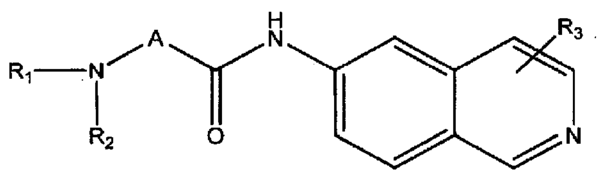

(1)

(in the formula, $R_1$ and $R_2$ each independently represent a hydrogen atom or a $C_1$-$C_4$ alkyl group,

$R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and

the formula (1) also includes a tautomer thereof)

or a salt thereof or a solvate thereof, the aqueous composition being housed in a container formed of a polyolefin resin.

Advantageous Effects of Invention

According to the present invention, it is possible to improve the light stability of isoquinolin-6-amino derivatives exemplified by netarsudil in an aqueous composition.

Brief Description of Drawings

[Fig. 1] Fig. 1 is a graph showing the transmittance (%) of the light having a wavelength in the range of from 270 to 800 nm of an eye drop container formed of polypropylene into which a benzotriazole-based ultraviolet absorbing agent was kneaded, the container being used in Test Example 3.

[Fig. 2] Fig. 2 is a graph showing the transmittance (%) of the light having a wavelength in the range of from 270 to 800 nm of an eye drop container formed of polypropylene and having a shrink film blended with an ultraviolet scattering agent, the container being used in Test Example 4.

Description of Embodiments

In the present specification, "a compound represented by the formula (1):

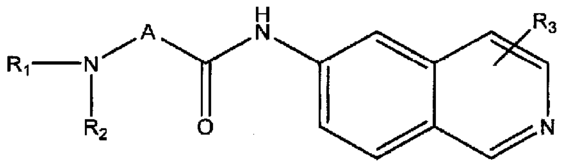

(1)

(in the formula, $R_1$ and $R_2$ each independently represent a hydrogen atom or a $C_1$-$C_4$ alkyl group,

$R_3$ represents a hydrogen atom or a hydroxy group, and
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof" includes not only a compound represented by the formula (1) itself but also a salt or solvate thereof.

Examples of the salt of the compound represented by the formula (1) are not particularly limited as long as those are pharmaceutically acceptable salts. Specific examples of the salt include an inorganic acid salt such as a hydrochloride, a sulfate, a nitrate, a hydrofluoride, or a hydrobromide; an organic acid salt such as an acetate, a tartrate, a lactate, a citrate, a fumarate, a maleate, a succinate, a methanesulfonate, an ethanesulfonate, a benzenesulfonate, a toluenesulfonate, a naphthalenesulfonate, or a camphorsulfonate, and the hydrochloride and the methanesulfonate are preferred.

Examples of the solvate of the compound represented by the formula (1) or a salt thereof include a hydrate and an alcohol solvate.

Furthermore, when an asymmetric carbon exists in a chemical structure of the compound represented by the formula (1), various stereoisomers can exist, but the configuration of the compound represented by the formula (1) is not particularly limited, and the compound may be a single stereoisomer or a mixture of various stereoisomers in any proportions.

In the specification, when an asymmetric carbon exists in a chemical structure of the compound represented by various formulae, the compound represented by the formula encompasses all of a variety of single stereoisomers and mixtures of the stereoisomers in any proportions, unless otherwise specifies the configuration. Therefore, compounds represented by the formula for which the configuration is not especially specified may be a single stereoisomer or a mixture of various stereoisomers in any proportions.

The compound represented by the formula (1) includes not only a compound directly represented by the formula (1), but also a tautomer of the compound. Specifically, for example, when the formula (1) is the following formula (1a) in which $R_3$ is a hydroxy group, a tautomer thereof (formula (1b)) can exist, then the "compound represented by the formula (1)" includes not only a compound represented by the following formula (1a), but also a compound represented by the formula (1b).

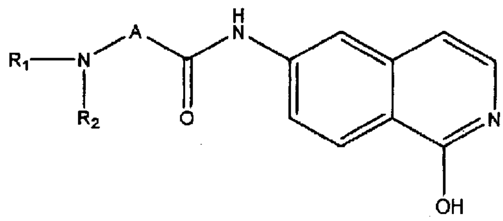

(1 a)

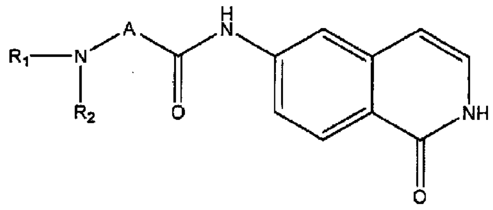

(1 b)

In the present specification, the "$C_1$-$C_4$ alkyl group" means a linear, branched or cyclic alkyl group having 1 to 4 carbon atoms. Specific examples of the "$C_1$-$C_4$ alkyl group" include a methyl group, an ethyl group, a n-propyl group, an isopropyl group, a cyclopropyl group, a n-butyl group, a t-butyl group, and an isobutyl group, and the methyl group is preferable.

In the present specification, the "$C_6$-$C_{10}$ aryl group" means an aryl group having 6 to 10 carbon atoms. Specific examples of the "$C_6$-$C_{10}$ aryl group" include a phenyl group and a naphthyl group, and the phenyl group is preferable.

In the present specification, the "5 to 10-membered heteroaryl group" means a 5 to 10-membered monocyclic, polycyclic or condensed cyclic aromatic heterocyclic group having 1 to 4 heteroatoms selected from the group consisting of an oxygen atom, a sulfur atom, and a nitrogen atom as a ring-constituting atom(s). Specific examples of the "5 to 10-membered heteroaryl group" include a furyl group, a thienyl group, a pyrrolyl group, an oxazolyl group, an isoxazolyl group, a thiazolyl group, an isothiazolyl group, an imidazolyl group, a pyrazolyl group, an oxadiazolyl group, a thiadiazolyl group, a triazolyl group, a tetrazolyl group, a pyridyl group, a pyrimidyl group, a pyrazinyl group, a pyridazinyl group, a benzofuranyl group, an isobenzofuranyl group, a benzothienyl group, an indolyl group, an isoindolyl group, an indazolyl group, a benzimidazolyl group, a benzoxazolyl group, a benzisoxazolyl group, a benzothiazolyl group, a benzisothiazolyl group, a benzoxadiazolyl group, a benzothiadiazolyl group, a benzotriazolyl group, a quinolyl group, an isoquinolyl group, a cinnolinyl group, a quinazolinyl group, a quinoxalinyl group, a phthalazinyl group, a naphthyridinyl group, a purinyl group, a pteridinyl group, a furopyridyl group, a thienopyridyl group, a pyrrolopyridyl group, an oxazolopyridyl group, a thiazolopyridyl group, and an imidazopyridyl group, and the thienyl group is preferable.

In the specification, examples of the "substituent" in the "optionally substituted $C_6$-$C_{10}$ aryl group" and "optionally substituted 5 to 10-membered heteroaryl group" specifically include a halogen atom and $-CH_2-OC(=O)-R_5$ (wherein, $R_5$ means a $C_6$-$C_{10}$ aryl group optionally having one to three $C_1$-$C_4$ alkyl group (s) as substituent(s) (e.g., 2,4-dimethylphenyl group), and a chlorine atom and a 2,4-dimethylphenyl carboxymethyl group are preferable.

In the specification, it is preferred that the "optionally substituted $C_6$-$C_{10}$ aryl group" is a 4-chlorophenyl group and a 4-(2,4-dimethylphenyl carboxymethyl)phenyl group.

In the specification, it is preferred that the "optionally substituted 5 to 10-membered heteroaryl group" is a 3-thienyl group.

When $R_3$ is a hydroxy group, the substitution position of $R_3$ is not limited as long as it is on the isoquinoline ring, but it is preferably position 1 of the isoquinoline ring.

In the specification, the "compound represented by the formula (1) or a salt thereof or a solvate thereof" is preferably a compound represented by the following formula (2):

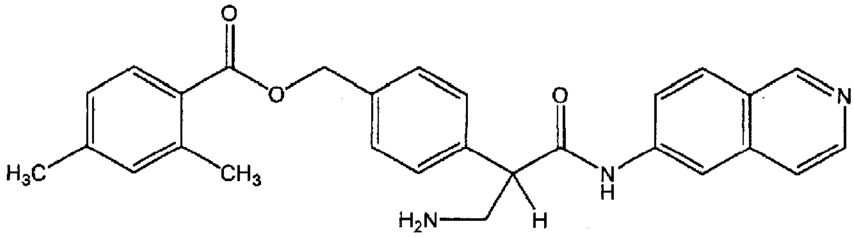

(2)

or a salt thereof or a solvate thereof; more preferably a compound represented by the following formula (3):

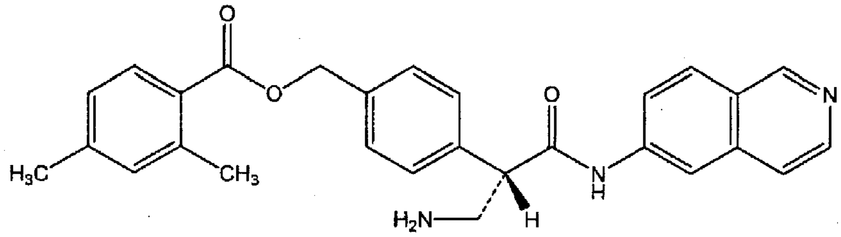

(3)

(netarsudil) (Chemical name: [4-[(2S)-3-amino-1-(isoquinolin-6-ylamino)-1-oxopropan-2-yl]phenyl]methyl 2,4-dimethylbenzoate), International Nonproprietary Name: netarsudil) or a salt thereof or a solvate thereof; and particularly preferably a compound represented by the following formula (4):

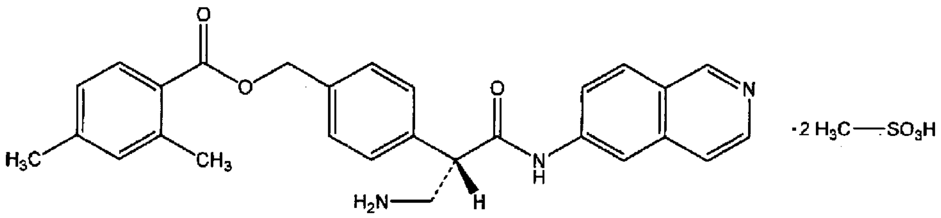

(4)

, which is a dimethanesulfonate of the compound represented by the formula (3), (chemical name: [4-[(2S)-3-amino-1-(isoquinolin-6-ylamino)-1-oxopropan-2-yl]phenyl]methyl 2,4-dimethylbenzoate dimethanesulfonate, hereinafter in the specification, it is sometimes referred to as "netarsudil dimesylate").

As another embodiment, the "compound represented by the formula (1) or a salt thereof or a solvate thereof" is preferably a compound represented by the following formula (5) or (5'):

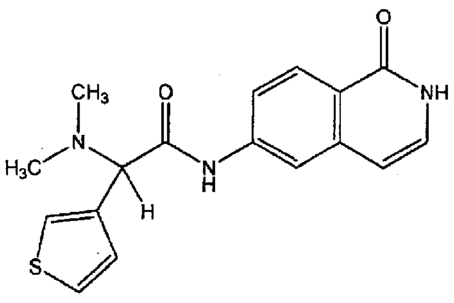

(5)

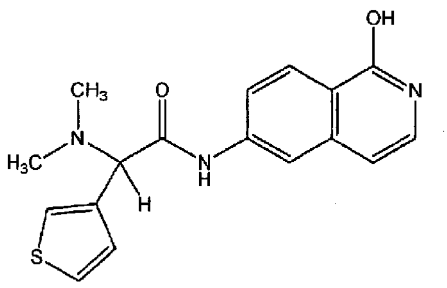

(5')

or a salt thereof or a solvate thereof; more preferably a compound represented by the following formula (6):

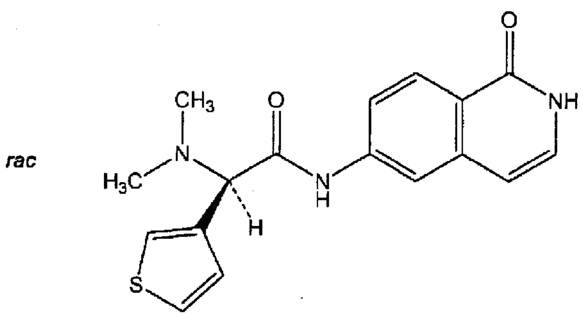

(6)

(verosudil) (Chemical name: rac-(2R)-2-(dimethylamino)-N-(1-oxo-1,2-dihydroisoquinolin-6-yl)-2-(thiophen-3-yl)acetamide), International Nonproprietary Name: verosudil), or a tautomer thereof or a salt of these or a solvate thereof;

and particularly preferably a compound represented by the following formula (7):

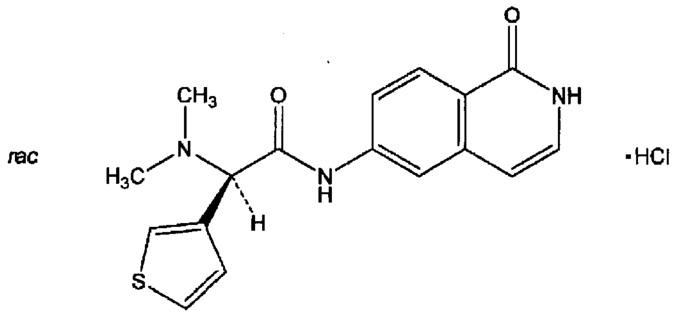

(7)

, which is a monohydrochloride of the compound represented by the formula (6), (chemical name: rac-(2R)-2-(dimethylamino)-N-(1-oxo-1,2-dihydroisoquinolin-6-yl)-2-(thiophen-3-yl)acetamide monohydrochloride, hereinafter in the specification, it is sometimes referred to as "verosudil monohydrochloride").

Further, as another embodiment, the "compound represented by the formula (1) or a salt thereof or a solvate thereof" is preferably a compound represented by the following formula (8):

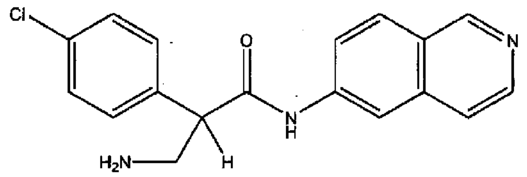

(8)

(3-amino-2-(4-chlorophenyl)-N-(isoquinolin-6-yl)propanamide) or a salt thereof or a solvate thereof.

The compound represented by the formula (1) or a salt thereof or a solvate thereof is known compounds, and they can be prepared by a known method. Specifically, for example, the compounds can be prepared with reference to the methods descried in Patent Literatures 1 to 3 or Non Patent Literature 2. The contents of these literatures are incorporated herein by reference.

The compound represented by the formula (1) or a salt thereof or a solvate thereof is commercially available, and these commercial products may be used. Specific examples of the commercial products include netarsudil dimesylate (the compound represented by the formula (4)) manufactured by MedChemExpress and by Chemscene LLS; netarsudil monohydrochloride (monohydrochloride of the compound represented by the formula (3)) manufactured by Shanghai Biopharmaleader Co., Ltd.; and verosudil (a free form of the compound represented by the formula (6)) manufactured by MedKoo Biosciences, Inc.

The content of the compound represented by the formula (1) or a salt thereof or a solvate thereof in the aqueous composition is not particularly limited, and can be appropriately determined depending on the applicable disease and patient's sex, age, symptoms or the like, but, from the viewpoint of obtaining an excellent pharmacological action, the content is preferably from 0.0001 to 5 w/v%, more preferably from 0.001 to 3 w/v%, still more preferably from 0.005 to 2 w/v %, and particularly preferably from 0.01 to 1 w/v% of the compound represented by the formula (1) in terms of a free form relative to the total volume of the aqueous composition.

Particularly, when netarsudil is used as the compound represented by the formula (1), the content is preferably from 0.0001 to 1 w/v%, more preferably from 0.001 to 0.5 w/v%, still more preferably from 0.005 to 0.1 w/v%, and particularly preferably from 0.01 to 0.04 w/v% of netarsudil in terms of a free form relative to the total volume of the aqueous composition, from the viewpoint of obtaining excellent pharmacological action.

Further, when verosudil is used as the compound represented by the formula (1), from the viewpoint of obtaining excellent pharmacological action, the content is preferably from 0.01 to 3.5 w/v%, more preferably from 0.1 to 2.5 w/v%, still more preferably from 0.2 to 1.5 w/v%, and particularly preferably from 0.3 to 0.8 w/v% of verosudil in terms of a free form relative to the total volume of the aqueous composition.

Furthermore, when the compound represented by the formula (8) is used as the compound represented by the formula (1), the content is preferably from 0.0001 to 3 w/v%, more preferably from 0.001 to 2 w/v%, still more preferably from 0.005 to 1 w/v%, and particularly preferably from 0.01 to 0.5 w/v% of the compound represented by the formula (8) in terms of a free form relative to the total volume of the aqueous composition, from the viewpoint of obtaining excellent pharmacological action.

In the present specification, the "aqueous composition" means a composition comprising at least water, and

the property of the aqueous composition is not particularly limited as long as it can be housed in a container described below, and for example, the aqueous composition may be in the form of liquid (solution or suspension), semi-solid (ointment), and the like. Examples of the water in the composition include purified water, water for injection, and sterilized purified water.

The content of water in the aqueous composition is not particularly limited, but preferably 5 % by mass or more, more preferably 20 % by mass or more, still more preferably 50 % by mass or more, even more preferably 90 % by mass or more, and particularly preferably from 90 to 99.99 % by mass.

The aqueous composition may further comprise, in addition to the compound represented by the formula (1) or a salt thereof or a solvate thereof, one or more components selected from the group consisting of an acid, a quaternary ammonium type surfactant, and a polyhydric alcohol.

As specifically shown in Test Examples 6 to 8 described below, when an aqueous composition comprising the compound represented by the formula (1) or a salt thereof or a solvate thereof is housed in a container made of a polyolefin resin, the content reduction of the compound represented by the formula (1) may occur by storage under high temperature conditions, which is presumptively attributed to the adsorption to the resin. However, it has been revealed that, by adding one or more components selected from the group consisting of an acid, a quaternary ammonium type surfactant and a polyhydric alcohol to the aqueous composition, the content reduction can be suppressed.

In the specification, the "acid" which may be suitably blended to the aqueous composition is not particularly limited, and the acid may be an organic or inorganic acid. Specific examples of the "acid" include "borate", "phosphate", and "aliphatic carboxylate". From the viewpoint of suppressing the content reduction of the compound represented by the formula (1), it is preferred to use one or more of the "borate", "phosphate" and "aliphatic carboxylate" appropriately in combination, and particularly preferred to use the "borate" as the "acid".

In this specification, the "borate" means one or more selected from the group consisting of boric acid and a salt thereof (e.g., an alkali metal salt such as a sodium salt or a potassium salt; an alkaline earth metal salt such as a calcium salt; and an ammonium salt) and a solvate thereof (e.g., a hydrate).

Specific examples of the borate include boric acid, ammonium borate, and borax. In the specification, it is preferred that the "borate" is boric acid.

All these borates are known compounds. They may be prepared by a known method, and it is also acceptable to use their commercial products.

In the present specification, the "phosphate" means one or more selected from the group consisting of phosphoric acid and a salt thereof (e.g., an alkali metal salt such as a sodium salt or a potassium salt; an alkaline earth metal salt such as a calcium salt; and an ammonium salt) and a solvate thereof (e.g., a hydrate).

Specific examples of the phosphate preferably include those listed in Pharmaceutical Excipients Dictionary 2016 (published by Yakuji Nippo) such as phosphoric acid, calcium monohydrogen phosphate, sodium monohydrogen phosphate heptahydrate, trisodium phosphate, dibasic calcium phosphate, dibasic calcium phosphate fine granulated, dibasic sodium phosphate, dibasic sodium phosphate heptahydrate, dibasic sodium phosphate dihydrate, disodium hydrogen phosphate dihydrate, dibasic potassium phosphate, monoabasic potassium phosphate, monobasic calcium phosphate hydrate, sodium dihydrogen phosphate dihydrate, sodium dihydrogen phosphate monohydrate, crystalline sodium dihydrogen phosphate dihydrate, calcium pyrophosphate, sodium pyrophosphate, anhydrous sodium pyrophosphate, disodium hydrogen phosphate anhydrous, anhydrous tribasic sodium phosphate, anhydrous dibasic calcium phosphate, anhydrous dibasic calcium phosphate fine granulated, sodium dihydrogen phosphate anhydrous, calcium polyphosphate, or sodium polyphosphate, and from the viewpoint of suppressing the content reduction of the compound represented by the formula (1), one or more selected from the group consisting of phosphoric acid, dibasic sodium phosphate, monobasic potassium phosphate, sodium dihydrogen phosphate dihydrate, sodium dihydrogen phosphate monohydrate, crystalline sodium dihydrogen phosphate dihydrate, disodium hydrogen phosphate anhydrous and sodium dihydrogen phosphate anhydrous are preferable.

All these phosphates are known compounds, so that they may be prepared by a known method, and it is acceptable to use their commercial products. Further, the phosphate in the form of a salt or a complex with another component may be used.

In the specification, the "aliphatic carboxylate" means one or more selected from the group consisting of aliphatic carboxylic acid and a salt thereof (e.g., an alkali metal salt such as a sodium salt or a potassium salt; an alkaline earth metal salt such as a calcium salt or a magnesium salt; and an ammonium salt) and a solvate thereof (e.g., a hydrate). Here, the number of carbon atoms in the aliphatic carboxylate is not particularly limited, but from the viewpoint of suppressing the content reduction of the compound represented by the formula (1), preferably from 2 to 12, more preferably from 2 to 6, and particularly preferably from 2 to 4. Further, the carbon chain may be linear or branched, and may be saturated or unsaturated. Additionally, the number of carboxyl groups in the aliphatic carboxylate is not particularly limited, but from the viewpoint of suppressing the content reduction of the compound represented by the formula (1), preferably from 1 to 4, more preferably from 2 to 4, and particularly preferably from 2 to 3.

The "aliphatic carboxylate" may be a carboxylic acid having an amino group as a substituent (amino acid), and

a carboxylic acid having a hydroxy group as a substituent (oxycarboxylic acid). In this case, the number of the substituent is not particularly limited, but preferably from 1 to 3, and particularly preferably from 1 to 2 from the viewpoint of suppressing the content reduction of the compound represented by the formula (1).

Specific examples of such aliphatic carboxylate include aspartic acid or a salt thereof or a solvate thereof such as sodium L-aspartate, potassium L-aspartate or magnesium L-aspartate; alanine or a salt thereof or a solvate thereof such as DL-alanine or L-alanine; arginine or a salt thereof or a solvate thereof such as L-arginine or L-arginine hydrochloride; epsilon-aminocaproic acid; edetic acid or a salt thereof or a solvate thereof such as calcium disodium edetate, sodium edetate hydrate, or tetrasodium edetate; citric acid or a salt thereof or a solvate thereof such as calcium citrate, citric acid hydrate, sodium citrate hydrate (trisodium citrate dihydrate), monobasic sodium citrate, dibasic sodium citrate, anhydrous citric acid, or sodium citrate anhydrous; glycine or a salt thereof or a solvate thereof; gluconic acid or a salt thereof or a solvate thereof such as gluconic acid, calcium gluconate hydrate, sodium gluconate, or magnesium gluconate; glutamine or a salt thereof or a solvate thereof; glutamic acid or a salt thereof or a solvate thereof such as L-glutamic acid, L-glutamic acid hydrochloride, potassium L-glutamate, or sodium L-glutamate; succinic acid or a salt thereof or a solvate thereof such as succinic acid, monosodium succinate, or disodium succinate hexahydrate; acetic acid or a salt thereof or a solvate thereof such as acetic acid, ammonium acetate, potassium acetate, calcium acetate, sodium acetate hydrate, glacial acetic acid, or anhydrous sodium acetate; cysteine or a salt thereof or a solvate thereof such as L-cystine, L-cysteine, or L-cysteine hydrochloride hydrate; tartaric acid or a salt thereof or a solvate thereof such as tartaric acid, D-tartaric acid, potassium hydrogen tartrate, sodium DL-tartrate, sodium L-tartrate, or sodium potassium tartrate; sorbic acid or a salt thereof or a solvate thereof such as sorbic acid or potassium sorbate; lactic acid or a salt thereof or a solvate thereof such as lactic acid, sodium lactate solution, calcium lactate hydrate or aluminum lactate; histidine or a salt thereof or a solvate thereof such as L-histidine or L-histidine hydrochloride hydrate; phenylalanine or a salt thereof or a solvate thereof; malonic acid or a salt thereof or a solvate thereof; methionine or a salt thereof or a solvate thereof such as DL-methionine or L-methionine; lysine or a salt thereof or a solvate thereof such as L-lysine or L-lysine hydrochloride; malic acid or a salt thereof or a solvate thereof such as malic acid, DL-malic acid or sodium DL-malate; and leucine or a salt thereof or a solvate thereof such as dl-leucine and L-leucine.

All these aliphatic carboxylic acids are known compounds, so that they may be prepared by a known method, or it is acceptable to use their commercial products. Further, the aliphatic carboxylate in the form of a salt or a complex with another component may be used.

The content of the acid in the aqueous composition is not particularly limited and can be appropriately determined, but from the viewpoint of suppressing the content reduction of the compound represented by the formula (1), it is preferably from 0.0001 to 4 w/v%, more preferably from 0.001 to 3 w/v%, and particularly preferably from 0.002 to 2.5 w/v% relative to the total volume of the aqueous composition. Particularly, in the case of using a borate as the acid, the content of the borate is not particularly limited and can be appropriately determined, but from the viewpoint of suppressing the content reduction of the compound represented by the formula (1), preferably from 0.003 to 2 w/v%, more preferably from 0.01 to 1 w/v%, and particularly preferably from 0.03 to 0.5 w/v% relative to the total volume of the aqueous composition.

The content mass ratio between the compound represented by the formula (1) or a salt thereof or a solvate thereof and the acid in the aqueous composition is not particularly limited, but from the viewpoint of suppressing the content reduction of the compound represented by the formula (1), the content of the acid is preferably from 0.0003 to 200 parts by mass, more preferably from 0.003 to 80 parts by mass, and particularly preferably from 0.02 to 30 parts by mass relative to 1 part by mass of the compound represented by the formula (1) or a salt thereof or a solvate thereof in terms of a free form. Above all, in the case of using a borate as the acid, the content mass ratio of the borate is not particularly limited, but from the viewpoint of suppressing the content reduction of the compound represented by the formula (1), the content of the borate is preferably from 0.0005 to 150 parts by mass, more preferably from 0.005 to 75 parts by mass, and particularly preferably from 0.03 to 25 parts by mass relative to 1 part by mass of the compound represented by the formula (1) or a salt thereof or a solvate thereof in terms of a free form.

In particular, in the case of using netarsudil as the compound represented by the formula (1) and using a borate as the acid, the content of the borate is, from the viewpoint of suppressing the content reduction of netarsudil, preferably from 0.5 to 100 parts by mass, more preferably from 1 to 50 parts by mass, and particularly preferably from 2 to 20 parts by mass relative to 1 part by mass of netarsudil or a salt thereof or a solvate thereof in terms of a free form.

Further, in the case of using verosudil as the compound represented by the formula (1) and using a borate as the acid, the content of the borate is, from the viewpoint of suppressing the content reduction of verosudil, preferably from 0.001 to 5 parts by mass, more preferably from 0.01 to 1 part by mass, and particularly preferably from 0.05 to 0.5 parts by mass relative to 1 part by mass of verosudil or a salt thereof or a solvate thereof in terms of a free form.

Furthermore, in the case of using the compound represented by the formula (8) as the compound represented by the formula (1) and using a borate as the acid, the content of the borate is, from the viewpoint of suppressing the content reduction of the compound represented by the formula (8), preferably from 0.01 to 80 parts by mass, more preferably from 0.05 to 40 parts by mass, and particularly preferably from 0.1 to 20 parts by mass relative to 1 part by

mass of the compound represented by the formula (8) or a salt thereof or a solvate thereof in terms of a free form.

In the specification, examples of the "quaternary ammonium type surfactant" which can be suitably blended in the aqueous composition include benzalkonium chloride, benzethonium chloride, and benzododecinium bromide, those are known as antiseptic. From the viewpoint of suppressing the content reduction of the compound represented by the formula (1), the quaternary ammonium type surfactant is preferably benzalkonium chloride or benzododecinium bromide, and particularly preferably benzalkonium chloride.

These quaternary ammonium type surfactants are known compounds, so that they may be prepared by a known method, or their commercial products may be used.

The content of the quaternary ammonium type surfactant in the aqueous composition is not particularly limited and may be appropriately determined, but from the viewpoint of suppressing the content reduction of the compound represented by the formula (1), preferably from 0.00005 to 0.3 w/v%, more preferably from 0.00025 to 0.2 w/v%, and particularly preferably from 0.00025 to 0.15 w/v% relative to the total volume of the aqueous composition. Above all, in the case of using benzalkonium chloride as the quaternary ammonium type surfactant, from the viewpoint of suppressing the content reduction of the compound represented by the formula (1), the content of the benzalkonium chloride is preferably from 0.0001 to 0.3 w/v%, more preferably from 0.0005 to 0.2 w/v%, and particularly preferably from 0.0005 to 0.15 w/v% relative to the total volume of the aqueous composition. Further, in the case of using benzododecinium bromide as the quaternary ammonium type surfactant, from the viewpoint of suppressing the content reduction of the compound represented by the formula (1), the content of the benzododecinium bromide is preferably from 0.0001 to 0.15 w/v%, more preferably from 0.0005 to 0.05 w/v%, and particularly preferably from 0.0005 to 0.02 w/v% relative to the total volume of the aqueous composition.

The content mass ratio between the compound represented by the formula (1) or a salt thereof or a solvate thereof in the aqueous composition and the quaternary ammonium type surfactant is not particularly limited, but from the viewpoint of suppressing the content reduction of the compound represented by the formula (1), the content of the quaternary ammonium type surfactant is preferably from 0.0005 to 8 parts by mass, more preferably from 0.005 to 7 parts by mass, and particularly preferably from 0.007 to 6 parts by mass relative to 1 part by mass of the compound represented by the formula (1) or a salt thereof or a solvate thereof in terms of a free form.

In particular, in the case of using netarsudil as the compound represented by the formula (1) and using benzalkonium chloride as the quaternary ammonium type surfactant, from the viewpoint of suppressing the content reduction of netarsudil, the content of the benzalkonium chloride is preferably from 0.05 to 5 parts by mass, more preferably from 0.1 to 4 parts by mass, and particularly preferably from 0.5 to 3 parts by mass relative to 1 part by mass of netarsudil or a salt thereof or a solvate thereof in terms of a free form.

Further, in the case of using verosudil as the compound represented by the formula (1) and using benzalkonium chloride as the quaternary ammonium type surfactant, from the viewpoint of suppressing the content reduction of verosudil, the content of the benzalkonium chloride is preferably from 0.001 to 1 part by mass, more preferably from 0.005 to 0.5 parts by mass, and particularly preferably from 0.01 to 0.3 parts by mass relative to 1 part by mass of verosudil or a salt thereof or a solvate thereof in terms of a free form.

Furthermore, in the case of using a compound represented by the formula (8) as the compound represented by the formula (1) and using benzalkonium chloride as the quaternary ammonium type surfactant, from the viewpoint of suppressing the content reduction of the compound represented by the formula (8), the content of the benzalkonium chloride is preferably from 0.05 to 5 parts by mass, more preferably from 0.1 to 4 parts by mass, and particularly preferably from 0.5 to 3 parts by mass relative to 1 part by mass of the compound represented by the formula (8) or a salt thereof or a solvate thereof in terms of a free form.

In the case of using netarsudil as the compound represented by the formula (1) and using benzododecinium bromide as the quaternary ammonium type surfactant, from the viewpoint of suppressing the content reduction of netarsudil, the content of the benzododecinium bromide is preferably from 0.07 to 5 parts by mass, more preferably from 0.2 to 4 parts by mass, and particularly preferably from 0.6 to 3 parts by mass relative to 1 part by mass of netarsudil or a salt thereof or a solvate thereof in terms of a free form.

Further, in the case of using verosudil as the compound represented by the formula (1) and using benzododecinium bromide as the quaternary ammonium type surfactant, from the viewpoint of suppressing the content reduction of verosudil, the content of the benzododecinium bromide is preferably 0.003 to 0.8 part by mass, more preferably 0.007 to 0.4 parts by mass, and particularly preferably 0.01 to 0.1 parts by mass relative to 1 part by mass of verosudil or a salt thereof or a solvate thereof in terms of a free form.

Furthermore, in the case of using a compound represented by the formula (8) as the compound represented by the formula (1) and using benzododecinium bromide as the quaternary ammonium type surfactant, from the viewpoint of suppressing the content reduction of the compound represented by the formula (8), the content of the benzododecinium bromide is preferably from 0.07 to 5 parts by mass, more preferably from 0.2 to 4 parts by mass, and particularly preferably from 0.4 to 3 parts by mass relative to 1 part by mass of a compound represented by the formula (8) or a salt thereof or a solvate thereof in terms of a free form.

In the specification, the "polyhydric alcohol" which can be suitably blended in the aqueous composition means an alcohol having two or more hydroxy groups in the same molecule, and examples of the polyhydric alcohol include glycerin; saccharide such as fructose or glucose; sugar alcohol such as sorbitol, mannitol or xylitol; trometamol; propylene glycol; and macrogol such as macrogol 400, macrogol 4000 or macrogol 6000. Among them, from the viewpoint of suppressing the content reduction of the compound represented by the formula (1), the polyhydric alcohol is preferably glycerin, sorbitol, propylene glycol, mannitol, or macrogol, and particularly preferably D-mannitol.

The polyhydric alcohols are known compounds, and they may be prepared by a known method or their commercial products may be used.

The content of the polyhydric alcohol in the aqueous composition is not particularly limited and can be appropriately determined, but from the viewpoint of suppressing the content reduction of the compound represented by the formula (1), preferably from 0.01 to 30 w/v%, more preferably from 0.1 to 20 w/v%, and particularly preferably from 0.5 to 5 w/v% relative to the total volume of the aqueous composition.

The content mass ratio between the compound represented by the formula (1) or a salt thereof or a solvate thereof and the polyhydric alcohol in the aqueous composition is not particularly limited, but from the viewpoint of suppressing the content reduction of the compound represented by the formula (1), the content of the polyhydric alcohol is preferably from 0.01 to 2500 parts by mass, more preferably from 0.05 to 1500 parts by mass, and particularly preferably from 0.5 to 750 parts by mass relative to 1 part by mass of the compound represented by the formula (1) or a salt thereof or a solvate thereof in terms of a free form.

In particular, in the case of using netarsudil as the compound represented by the formula (1), from the viewpoint of suppressing the content reduction of netarsudil, the content of polyhydric alcohol is preferably from 10 to 2000 parts by mass, more preferably from 20 to 1000 parts by mass, and particularly preferably from 30 to 500 parts by mass relative to 1 part by mass of netarsudil or a salt thereof or a solvate thereof in terms of a free form.

Further, in the case of using verosudil as the compound represented by the formula (1), from the viewpoint of suppressing the content reduction of verosudil, the content of polyhydric alcohol is preferably from 0.1 to 200 parts by mass, more preferably from 0.5 to 100 parts by mass, and particularly preferably from 1 to 50 parts by mass relative to 1 part by mass of verosudil or a salt thereof or a solvate thereof in terms of a free form.

Furthermore, in the case of using a compound represented by the formula (8) as the compound represented by the formula (1), from the viewpoint of suppressing the content reduction of the compound represented by the formula (8), the content of polyhydric alcohol is preferably from 10 to 2000 parts by mass, more preferably from 20 to 1000 parts by mass, and particularly preferably from 30 to 500 parts by mass relative to 1 part by mass of the compound represented by the formula (8) or a salt thereof or a solvate thereof in terms of a free form.

The aqueous composition may comprise one or more additives utilized in pharmaceutical products, quasi-drugs and the like, besides the components described above, depending on the dosage form. Examples of the additives include an inorganic salt, an isotonic agent, a chelating agent, a stabilizing agent, a pH adjusting agent, a preservative, an antioxidant, a thickening agent, a surfactant, a solubilizer, a suspending agent, a refreshing agent, a dispersing agent, a preserving agent, an oily base, an emulsion base, and a water-soluble base. The "acid", "quaternary ammonium type surfactant" or "polyhydric alcohol" described above may be used as the additive.

Specific examples of the additive include ascorbic acid, potassium aspartate, sodium bisulfite, alginic acid, sodium benzoate, benzyl benzoate, fennel oil, ethanol, an ethylene-vinyl acetate copolymer, potassium chloride, calcium chloride hydrate, sodium chloride, magnesium chloride, alkyldiaminoethylglycine hydrochloride solution, a carboxyvinyl polymer, dried sodium sulfite, dried sodium carbonate, d-camphor, dl-camphor, creatinine, chlorobutanol, geraniol, sodium chondroitin sulfate, titanium oxide, gellan gum, dibutylhydroxytoluene, potassium bromide, sodium hydroxide, polyoxyl 45 stearate, purified lanolin, taurine, sodium hydrogen carbonate, sodium carbonate hydrate, sodium thiosulfate hydrate, thimerosal, tyloxapol, sodium dehydroacetate, a concentrated mixed tocopherol, white petrolatum, peppermint water, peppermint oil, ethyl parahydroxybenzoate, butyl parahydroxybenzoate, propyl parahydroxybenzoate, methyl parahydroxybenzoate, sodium hyaluronate, human serum albumin, hydroxyethyl cellulose, hydroxypropyl cellulose, hypromellose, sodium pyrosulfite, phenyl ethyl alcohol, bergamot oil, benzyl alcohol, povidone, polyoxyethylene (200) polyoxypropylene glycol (70), sodium polystyrenesulfonate, polysorbate 80, polyoxyethylene hydrogenated castor oil 60, d-borneol, methanesulfonic acid, methyl cellulose, 1-menthol, monoethanolamine, aluminum monostearate, polyethylene glycol monostearate, eucalyptus oil, potassium iodide, oxyquinoline sulfate, liquid paraffin, borneol, and vaseline.

Among the above, for example, potassium chloride, calcium chloride hydrate, sodium chloride, magnesium chloride, sodium hydroxide, sodium hydrogen carbonate, sodium carbonate hydrate, hydroxyethyl cellulose, hydroxypropyl cellulose, hypromellose, povidone, polysorbate 80, polyoxyethylene hydrogenated castor oil, polyethylene glycol monostearate, methyl cellulose, monoethanolamine, sodium hyaluronate, or 1-menthol is preferred as the additive.

The aqueous composition may further comprise, in addition to the components described above, one or more other medicinal components, depending on the applicable disease or the like. Examples of the medicinal components include $\alpha$1 receptor blockers including bunazosin or a salt thereof or a solvate thereof such as bunazosin hydrochloride;

α2 receptor agonists including brimonidine or a salt thereof or a solvate thereof such as brimonidine tartrate, and apraclonidine or a salt thereof or a solvate thereof; β-blockers including carteolol or a salt thereof or a solvate thereof such as carteolol hydrochloride, nipradilol or a salt thereof or a solvate thereof, timolol or a salt thereof or a solvate thereof such as timolol maleate, betaxolol or a salt thereof or a solvate thereof such as betaxolol hydrochloride, levobunolol or a salt thereof or a solvate thereof such as levobunolol hydrochloride, befunolol or a salt thereof or a solvate thereof, and metipranolol or a salt thereof or a solvate thereof; carbonic anhydrase inhibitors including dorzolamide or a salt thereof or a solvate thereof such as dorzolamide hydrochloride, brinzolamide or a salt thereof or a solvate thereof, acetazolamide or a salt thereof or a solvate thereof, dichlorphenamide or a salt thereof or a solvate thereof, and methazolamide or a salt thereof or a solvate thereof; prostaglandins and their derivatives (e.g., prostaglandin F2α derivatives) including isopropyl unoprostone or a salt thereof or a solvate thereof, tafluprost or a salt thereof or a solvate thereof, travoprost or a salt thereof or a solvate thereof, bimatoprost or a salt thereof or a solvate thereof, latanoprost or a salt thereof or a solvate thereof, cloprostenol or a salt thereof or a solvate thereof, and fluprostenol or a salt thereof or a solvate thereof; Rho kinase inhibitors including Ripasudil or a salt thereof or a solvate thereof, Y-39983, and H-1129; sympathomimetic drugs including dipivefrine or a salt thereof or a solvate thereof such as dipivefrin hydrochloride, and epinephrine or a salt thereof or a solvate thereof such as epinephrine, epinephrine borate, or epinephrine hydrochloride; parasympathomimetic drugs including distigmine bromide or a salt thereof or a solvate thereof, pilocarpine or a salt thereof or a solvate thereof such as pilocarpine, pilocarpine hydrochloride or pilocarpine nitrate, and carbachol or a salt thereof or a solvate thereof; calcium antagonists including lomerizine or a salt thereof or a solvate thereof such as lomerizine hydrochloride; and cholinesterase inhibitors including demecarium or a salt thereof or a solvate thereof, echothiophate or a salt thereof or a solvate thereof, and physostigmine or a salt thereof or a solvate thereof. These medicinal components can be blended singly or a combination of two or more.

As the other medicinal components, β-blockers are preferred, and especially timolol is preferred.

The aqueous composition may be one which does not contain the prostaglandin and any derivative thereof. For example, one embodiment of the aqueous composition is preferably other than the following <A-1> to <A-10>:

<A-1> a composition comprising (rac)-2-(dimethylamino)-N-(1-hydroxy-isoquinolin-6-yl)-2-(thiophen-3-yl)acetamide hydrochloride, travoprost, boric acid, D-mannitol, benzalkonium chloride, polyoxyl 40 stearate, polyethylene glycol 400, EDTA, and purified water.

<A-2> a composition comprising (rac)-2-(dimethylamino)-N-(1-hydroxy-isoquinolin-6-yl)-2-(thiophen-3-yl)acetamide hydrochloride, travoprost, boric acid, D-mannitol, benzalkonium chloride, Cremophor RH40, polyethylene glycol 400, EDTA, and purified water.

<A-3> a composition comprising (rac)-2-(dimethylamino)-N-(1-hydroxy-isoquinolin-6-yl)-2-(thiophen-3-yl)acetamide hydrochloride, travoprost, boric acid, D-mannitol, polyoxyl 40 stearate, polyethylene glycol 400, EDTA, and purified water.

<A-4> a composition comprising (rac)-2-(dimethylamino)-N-(1-hydroxy-isoquinolin-6-yl)-2-(thiophen-3-yl)acetamide hydrochloride, latanoprost, monobasic sodium phosphate, dibasic sodium phosphate, benzalkonium chloride, sodium chloride, EDTA, and purified water.

<A-5> a composition comprising (rac)-2-(dimethylamino)-N-(1-hydroxy-isoquinolin-6-yl)-2-(thiophen-3-yl)acetamide hydrochloride, latanoprost, boric acid, D-mannitol, benzalkonium chloride, EDTA, and purified water.

<A-6> a composition comprising (rac)-2-(dimethylamino)-N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.

<A-7> a composition comprising (rac)-2-(dimethylamino)-N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.

<A-8> a composition comprising (rac)-2-(dimethylamino)-N-(1-hydroxy-isoquinolin-6-yl)-2-(thiophen-3-yl)acetamide hydrochloride, bimatoprost, boric acid, D-mannitol, and purified water.

<A-9> a composition comprising (S)-4-(3-amino-1-(isoquinolin-6-ylamino)-1-oxopropan-2-yl)benzyl 2,4-dimethylbenzoate, travoprost, boric acid, D-mannitol, benzalkonium chloride, polyoxyl 40 stearate, polyethylene glycol 400, EDTA, and purified water.

<A-10> a composition comprising (S)-4-(3-amino-1-(isoquinolin-6-ylamino)-1-oxopropan-2-yl)benzyl 2,4-dimethylbenzoate, latanoprost, boric acid, D-mannitol, benzalkonium chloride, polyoxyl 40 stearate, polyethylene glycol 400, EDTA, and purified water.

The pH of the aqueous composition (at 25°C) is not particularly limited, but preferably from 3 to 9, more preferably from 3.5 to 8, still more preferably from 4 to 7, particularly preferably 5 to 6. Further, the osmotic pressure ratio of the aqueous composition to physiological saline is not particularly limited, but preferably from 0.6 to 3, particularly preferably from 0.6 to 2.

The pharmaceutical preparation of the present invention uses a polyolefin resin container.

As used herein, the "container" means a package for directly housing the aqueous composition. The concept of the container encompasses any of "well-closed container", "tight container" and "hermetic container" which are defined in the Japanese Pharmacopoeia, Seventeenth Edition, General Notices.

The form of the container is not particularly limited as long as it is capable of housing the aqueous composition, and may be appropriately selected and set depending on the dosage form, the application of the pharmaceutical preparation, and the like. Specific examples of the container in such a form include a container for injection, a container for inhalation, a container for spray, a bottle-shaped container, a tube-shaped container, an eye drop container, a nasal drop container, an ear drop container, a bag shaped container, and the like.

The container, from the viewpoint of advantageously utilizing pharmacological action of the compound represented by the formula (1), is preferably an eye drop container.

In the present specification, the "container formed of a polyolefin resin" means a container of which at least a part in contact with the aqueous composition is "formed of a polyolefin resin". Thus, for example, a container having a polyolefin resin layer as an inner layer in contact with an aqueous composition, and further having a layer of other resin material laminated on the outside of the inner layer also corresponds to the "polyolefin resin container". The polyolefin resin is not particularly limited, and it may be a polymer composed of single kind of monomer (homopolymer) or a copolymer composed of plural kinds of monomers (copolymer). In the case of a copolymer, the polymerization mode is not particularly limited, and the copolymer may be a random copolymer or a block copolymer. Furthermore, the stereoregularity (tacticity) of the copolymer is not particularly limited.

Specific examples of the polyolefin resin include polyethylene (more specifically including low density polyethylene (linear low density polyethylene), high density polyethylene, medium density polyethylene), polypropylene, cyclic polyolefin, poly(4-methylpentene), polytetrafluoroethylene, an ethylene-propylene copolymer, an ethylene-$\alpha$-olefin copolymer, an ethylene-acrylic acid copolymer, an ethylene-methacrylic acid copolymer, an ethylene-vinyl acetate copolymer, and an ethylene-ethyl acrylate copolymer. These resins can be used singly or in a combination of two or more. From the viewpoint of suppressing the content reduction of the compound represented by the formula (1) due to exposure to light or storage under high temperature, the polyolefin resin is preferably, polyethylene, polypropylene or cyclic polyolefin, more preferably, polyethylene or polypropylene, and particularly preferably, polypropylene.

In the present specification, the phrase "formed of a polyolefin resin" means that the material comprises, at least in part, a polyolefin resin. For example, the one formed of a mixture of two or more kinds of resin (polymer alloy) of a polyolefin resin and one or more other kinds of resin also correspond to the one "formed of a polyolefin resin".

The container formed of a polyolefin resin may be one blocking a light of the certain wavelength range.

As specifically shown in Test Examples 3 to 4, it has been revealed that, by housing the aqueous composition comprising the compound represented by the formula (1) or a salt thereof or a solvate thereof in a container formed of a polyolefin resin blocking a light having a wavelength of from 320 to 380 nm, the content reduction of the compound represented by the formula (1) in the aqueous composition due to exposure to light can be further suppressed.

The wavelength range of the light to be blocked by the container formed of a polyolefin resin is sufficient if it is within a range of from 320 to 380 nm (preferably from 320 to 395 nm) in view of normal storage environment of the pharmaceutical preparation. However, from the viewpoint of further improving the light stability, the wavelength range of the light to be blocked is preferably from 300 to 380 nm, more preferably from 300 to 395 nm. Further, considering the use under storage conditions where it is likely to be exposed to wavelength of particularly below 300 nm, the wavelength range of the light to be blocked is preferably from 270 to 380 nm, particularly preferably from 270 to 395 nm.

In the present specification, the terms "blocking a light" of the aforesaid wavelength range means that the average value of the transmittance of the light of the aforesaid wavelength range is 40% or less. Accordingly, for example, the "container which blocks a light having a wavelength of from 320 to 380 nm" means a container of which the average value of the transmittance of the light having a wavelength of from 320 to 380 nm is 40% or less.

From the viewpoint of further improving light stability, the average value of the transmittance of the light of the aforesaid wavelength range of the container is preferably 35% or less, more preferably 30% or less, still more preferably 25% or less, even more preferably 20% or less, still even more preferably 15% or less, furthermore preferably 10% or less, and particularly preferably 5% or less.

In the present specification, the measurement of an average value of the transmittance of the light in a certain wavelength range can be carried out by measuring the transmittance of the light of the container in air in the certain wavelength range at 5 nm intervals using a spectrophotometer, and then calculating the average value of the measured transmittance. Examples of the spectrophotometer include U-3900 (Hitachi High-Technologies Corporation).

By housing the aqueous composition in such a container formed of a polyolefin resin which blocks a light as described above, specifically in the case where the aqueous composition is irradiated with light so that the cumulative irradiation dose at a temperature of 25$\pm$5°C is 1.2 million lux·hr using a D65 fluorescent lamp as a light source, it is possible to make the residual rate of the compound represented by the formula (1) in the aqueous composition after irradiation be, for example, 80% or more (preferably 85% or more, more preferably 90% or more, particularly preferably

95% or more). The residual rate of the compound represented by the formula (1) in the aqueous composition can be obtained by measuring the concentrations of the compound in the aqueous composition before and after light irradiation, respectively, and calculating the residual rate by the following expression using the measured concentrations.

Residual rate (%) of the compound represented by the formula (1) = {(Concentration of the compound represented by the formula (1) in the aqueous composition after light irradiation)/(Concentration of the compound represented by the formula (1) in the aqueous composition before light irradiation)} × 100

Specific measures to impart the property of blocking light in the aforesaid wavelength range to the container formed of a polyolefin resin are not particularly limited, but examples of the methods include a method using a substance which blocks a light of the aforesaid wavelength of range. More specific examples of the method include:

a method of adding a substance which blocks a light of the aforesaid wavelength range to the container formed of a polyolefin resin (for example, a method comprising adding a substance which blocks a light of the aforesaid wavelength range to the polyolefin resin and then forming the obtained resin into a container shape);
a method for providing a member (for example, a film) comprising a substance which blocks a light of the aforesaid wavelength range on the surface (at least one surface of the inside and outside surfaces) of a container formed of a polyolefin resin (for example, a method of adding a substance which blocks a light of the aforesaid wavelength range to a resin and forming the obtained resin into a heat shrink film, and then winding the film on the outside surface of the container); and
a method involving applying a substance which blocks a light of the aforesaid wavelength range to the surface (at least one surface of the inside and outside surfaces) of a container formed of a polyolefin resin.

The substance which blocks a light of the aforesaid wavelength range is not particularly limited, but from the viewpoint of allowing the inside of the container formed of a polyolefin resin to be visible, preferably a substance which interferes with transmission of ultraviolet light, such as an ultraviolet absorbing agent or an ultraviolet scattering agent. Specific examples of the ultraviolet scattering agent include titanium oxide and zinc oxide. Examples of the ultraviolet absorbing agent include: benzotriazole-based ultraviolet absorbent such as 2-(2H-benzotriazol-2-yl)-p-cresol (e.g., Tinuvin P: BASF), 2-(2H-benzotriazol-2-yl)-4,6-bis(1-methyl-1-phenylethyl)phenol (e.g., Tinuvin 234: BASF), 2-(3,5-di-tert-butyl-2-hydroxyphenyl)benzotriazole (e.g., Tinuvin 320: BASF), 2-[5-chloro-(2H)-benzotriazol-2-yl]-4-methyl-6-t-butylphenol (e.g., Tinuvin 326: BASF), 2-(3,5-di-t-butyl-2-hydroxyphenyl)-5-chloro benzotriazole (e.g., Tinuvin 327: BASF), 2-(2H-benzotriazol-2-yl)-4,6-di-tert-pentylphenol (e.g., Tinuvin PA328: BASF), 2-(2H-benzotriazol-2-yl)-4-(1,1,3,3-tetramethylbutyl)phenol (e.g., Tinuvin 329: BASF), 2,2'-methylenebis[6-(2H-benzotriazol-2-yl)-4-(1,1,3,3-tetramethylbutyl)phenol (e.g., Tinuvin 360: BASF), a reaction product of methyl 3-(3-(2H-benzotriazol-2-yl)-5-tert-butyl-4-hydroxyphenyl)propionate and polyethylene glycol 300 (e.g., Tinuvin 213: BASF), 2-(2H-benzotriazol-2-yl)-6-dodecyl-4-methylphenol (e.g., Tinuvin 571: BASF), 2-(2'-hydroxy-3',5'-di-t-amylphenyl)benzotriazole, 2-[2'-hydroxy-3'-(3",4",5",6"-tetrahydrophthalimidemethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbing agent such as 2,2-bis{[2-cyano-3,3-diphenylacryloyloxy]methyl}propane-1,3-diyl=bis(2-cyano-3,3-diphenylacrylate) (for example, Uvinul 3030 FF: BASF), ethyl 2-cyano-3,3-diphenylacrylate (e.g., Uvinul 3035: BASF), and 2-ethylhexyl 2-cyano-3,3-diphenylacrylate (e.g., Uvinul 3039: BASF) and others; triazine-based ultraviolet absorbing agent such as 2-(4,6-diphenyl-1,3,5-triazine-2-yl)-5-[(hexyl)oxy]-phenol (e.g., Tinuvin 1577 ED: BASF); benzophenone-based ultraviolet absorbing agent such as octabenzone (e.g., Chimassorb 81: BASF), 2,2'-dihydroxy-4,4'-dimethoxybenzophenone (e.g., Uvinul 3049: BASF), 2,2'-4,4'-tetrahydrobenzophenone (e.g., Uvinul 3050: BASF), oxybenzone, hydroxymethoxybenzophenone sulfonic acid, sodium hydroxy methoxy benzophenone sulfonate, dihydroxydimethoxybenzophenone, sodium dihydroxydimethoxybenzophenone disulfonate, dihydroxybenzophenone, and tetrahydroxybenzophenone; cinnamic acid-based ultraviolet absorbing agent such as methyl diisopropyl cinnamate, cinoxate, glyceryl mono-2-ethylhexanoate diparamethoxycinnamate, a mixture of isopropyl paramethoxycinnamate and diisopropyl cinnamate, 2-ethylhexyl paramethoxycinnamate, and benzyl cinnamate; benzoic acid ester-based ultraviolet absorbing agent such as para-aminobenzoic acid, ethyl para-aminobenzoate, glyceryl para-aminobenzoate, amyl para-dimethylaminobenzoate, 2-ethylhexyl para-dimethylaminobenzoate, and ethyl 4-[N,N-di(2-hydroxypropyl)amino]benzoate; salicylic acid-based ultraviolet absorbing agent such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino]1,3,5-triazine; para-hydroxyanisole; 4-tert-butyl-4'-methoxydibenzoylmethane; phenylbenzimidazole sulfonate; and hexyl 2-(4-diethylamino-2-hydroxybenzoyl)benzoate. As a substance which blocks a light of the aforesaid wavelength range, these substances may be used singly or in a suitable combination of two or more so as to block the light of the aforesaid wavelength range. The substance which blocks a light of the aforesaid wavelength range is preferably titanium oxide and benzotriazole-based ultraviolet absorbing agent.

When adding the substance which blocks a light of the aforesaid wavelength range to the material or member of the container formed of a polyolefin resin, the blending ratio is determined depending on the kind of the substance, the material of the container or additional member and the like, but, for example, it may be about from 0.001 to 50 % by mass, preferably from 0.002 to 25 % by mass, particularly preferably from 0.01 to 10 % by mass relative to the material or additional member of the container formed of a polyolefin resin.

In this specification, the "container which blocks a light" includes a container in which only a portion of the container blocks a light. In such a container, based on the inner surface of the container, the portion which blocks a light is preferably 10% or more portions, more preferably 30% or more portions, still more preferably 50% or more portions, and particularly preferably 70% or more portions relative to the total area of the inner surface.

It is preferred that the inside of the container formed of a polyolefin resin is visible (observable) to the naked eye or the like. When the inside is visible, there are some advantages that it becomes possible to examine the presence or absence of contamination or the like in the manufacturing process of the pharmaceutical preparation; and that the user of the pharmaceutical preparation can check the remained content (aqueous composition), and the like.

In this specification, the "inside is visible" means a state that the inside of the container is visible from at least a portion of the outer surface of the container. (For example, an eye drop container, which usually has a nearly cylindrical shape, having a bottom surface allowing the inside of the container to be visible corresponds to the container whose "inside is visible", even when the inside of the container is not visible from the side surface owing to a shrink film or the like.)

The visibility is sufficient if the container has a transparency of no less than a certain level, and specifically, for example, it is sufficient that the average value of the transmittance of the light in the visible light region (450 to 750 nm) is around 30% or more (more suitably around 40% or more, and particularly suitably around 50% or more), but the visibility is not limited thereto.

Specific embodiments of the container formed of a polyolefin resin include the following embodiments:

1) a container (preferably, a container whose inside is visible) formed of a polyolefin resin (preferably, polypropylene) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent);

2) a container (preferably, a container whose inside is visible) formed of a polyolefin resin (preferably, polypropylene) having a member (preferably, a heat-shrinkable film (a shrink film)) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent), the member being wound around on a side surface of the container.

The means for housing the aqueous composition in the container formed of a polyolefin resin is not particularly limited and it can be performed by filling or the like with an ordinary method depending on the form of the container or the like.

The pharmaceutical preparation or aqueous composition can be formulated in various dosage forms according to a known method described in the Japanese Pharmacopoeia, Seventeenth Edition, General Rules for Preparation or the like. Examples of the dosage form include injection, inhalation liquid, eye drop, eye ointment, ear drop, nasal drop liquid, enema formulation, topical liquid, spray, ointment, gel, oral liquid, and syrup. From the viewpoint of advantageously utilizing the pharmacological actions of the compound represented by the formula (1), the dosage form is preferably, a dosage form for eye disease, specifically, eye drop or eye ointment, and particularly preferably, eye drop.

The applicable disease of the pharmaceutical preparation is not particularly limited, and it is appropriately selected depending on the pharmacological actions of the compound represented by the formula (1) or the like.

Specifically, for example, the pharmaceutical preparation can be used as a prevention or treatment agent for ocular hypertension and glaucoma, based on Rho kinase inhibitory activity, norepinephrine transporter inhibitory activity, and intraocular pressure-decreasing activity of the compound represented by the formula (1). Detailed examples of the

glaucoma include primary open-angle glaucoma, normal tension glaucoma, hypersecretion glaucoma, acute angle closure glaucoma, chronic angle closure glaucoma, plateau iris syndrome, mixed-type glaucoma, steroid glaucoma, glaucoma capsulare, pigment glaucoma, amyloid glaucoma, angiogenesis glaucoma, and malignant glaucoma.

In the case of using the aqueous composition or pharmaceutical preparation as a prevention or treatment agent for eye disease (appropriately, a diseases selected from the group of ocular hypertension and glaucoma), the aqueous composition or pharmaceutical preparation may be administered, for example, about 1 to 3 times a day in a suitable dose.

Hereinafter, examples of embodiments or aspects of the present invention are disclosed below; however, the present invention is not limited thereto.

[1] A pharmaceutical preparation comprising an aqueous composition comprising a compound represented by the following formula (1)

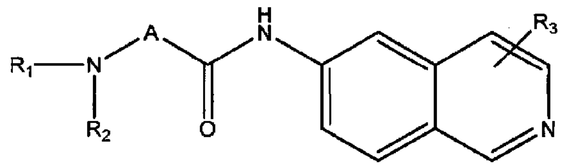

(1)

(in the formula, $R_1$ and $R_2$ each independently represent a hydrogen atom or a $C_1$-$C_4$ alkyl group,

$R_3$ represents a hydrogen atom or a hydroxy group, and
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof, the aqueous composition being housed in a container formed of a polyolefin resin.

[2] The pharmaceutical preparation according to [1], wherein the compound represented by the formula (1) is a compound represented by the following formula (2), (5), (5') or (8) :

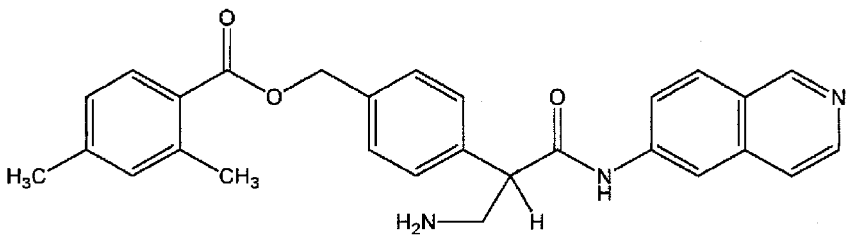

(2) ;

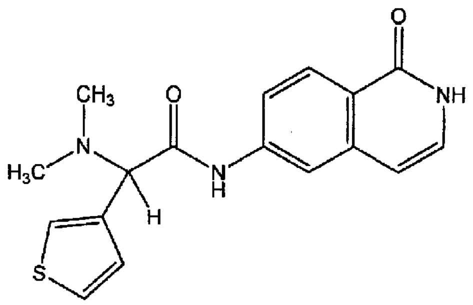

(5) ;

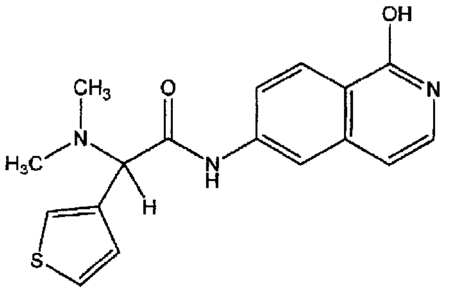

(5') ;

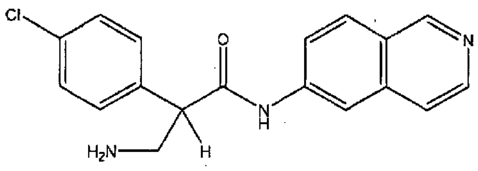

(8) .

[3] The pharmaceutical preparation according to [1], wherein the compound represented by the formula (1) is a compound represented by the following formula (3) or (6):

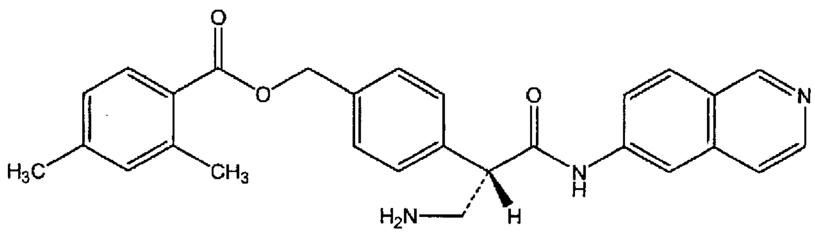

(3) ;

rac

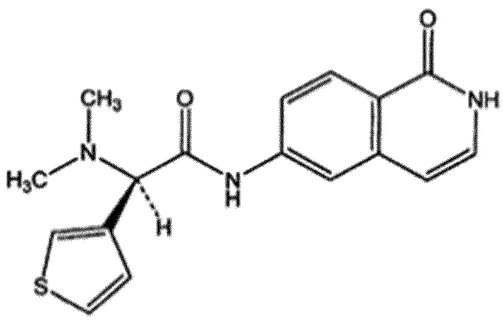

(6) .

[4] The pharmaceutical preparation according to [1], wherein the compound represented by the formula (1) is a compound represented by the following formula (4) or (7):

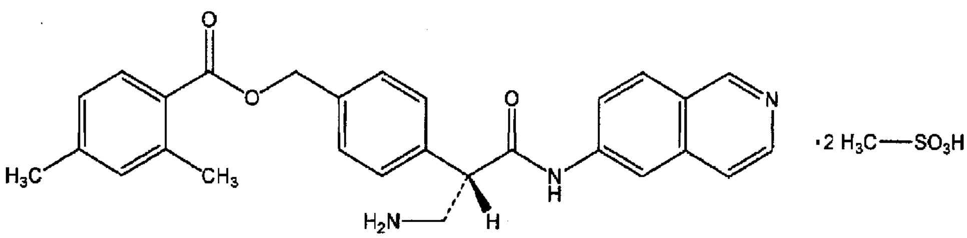

(4) ;

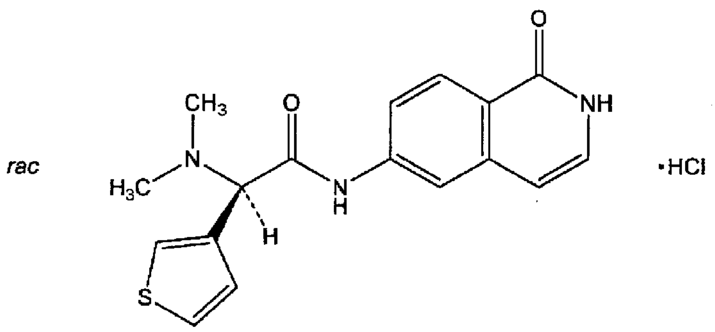

(7) .

[5] The pharmaceutical preparation according to any one of [1] to [4], wherein the polyolefin resin is polyethylene or polypropylene.
[6] The pharmaceutical preparation according to any one of [1] to [5], wherein the aqueous composition further comprises one or more components selected from the group consisting of an acid, a quaternary ammonium type surfactant, and a polyhydric alcohol.
[7] The pharmaceutical preparation according to any one of [1] to [6], wherein the container is a container which blocks a light having a wavelength of from 320 to 380 nm (preferably from 320 to 395 nm).
[8] The pharmaceutical preparation according to any one of [1] to [6], wherein the container is a container which blocks a light having a wavelength of from 300 to 380 nm (preferably from 300 to 395 nm).
[9] The pharmaceutical preparation according to any one of [1] to [6], wherein the container is a container which blocks a light having a wavelength of from 270 to 380 nm (preferably from 270 to 395 nm).
[10] The pharmaceutical preparation according to any one of [1] to [9], wherein a residual rate of the compound represented by the formula (1) in the aqueous composition after irradiation is 80% or more (preferably 85% or more, more preferably 90% or more, still more preferably 95% or more, and particularly preferably 97% or more), when the aqueous composition is irradiated with a light so that a cumulative irradiation dose at a temperature of 25$\pm$5°C is 1.2 million lux·hr using a D65 fluorescent lamp as a light source.
[11] The pharmaceutical preparation according to any one of [1] to [10], wherein the inside of the container is visible.
[12] The pharmaceutical preparation according any one of [1] to [11], wherein the container is 1) or 2) as follows:

1) a container (preferably, a container whose inside is visible) formed of a polyolefin resin (preferably, polypropylene) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent);
2) a container (preferably, a container whose inside is visible) formed of a polyolefin resin (preferably, polypropylene) having a member (preferably, a heat-shrinkable film (a shrink film)) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent), the member being wound around on a side surface of the container.

[13] A method for improving light stability of a compound represented by the following formula (1) or a salt thereof or a solvate thereof in an aqueous composition, comprising a step of housing the aqueous composition comprising the compound represented by the formula (1) or a salt thereof or a solvate thereof in a container formed of a polyolefin resin.
[14] The method according to [13], wherein the compound represented by the formula (1) is a compound represented by the formula (2), (5), (5') or (8).
[15] The method according to [13], wherein the compound represented by the formula (1) is a compound represented by the formula (3) or (6).
[16] The method according to [13], wherein the compound represented by the formula (1) is a compound represented by the formula (4) or (7).
[17] The method according to any one of [13] to [16], wherein the polyolefin resin is polyethylene or polypropylene.
[18] The method according to any one of [13] to [17], wherein the container is a container which blocks a light having a wavelength of from 320 to 380 nm (preferably from 320 to 395 nm).
[19] The method according to any one of [13] to [17], wherein the container is a container which blocks a light having a wavelength of from 300 to 380 nm (preferably from 300 to 395 nm).

[20] The method according to any one of [13] to [17], wherein the container is a container which blocks a light having a wavelength of from 270 to 380 nm (preferably from 270 to 395 nm).
[21] The method according to any one of [13] to [20], wherein the residual rate of the compound represented by the formula (1) in the aqueous composition after irradiation is 80% or more (preferably 85% or more, more preferably 90% or more, still more preferably 95% or more, and particularly preferably 97% or more), when the aqueous composition is irradiated with a light so that a cumulative irradiation dose at a temperature of 25$\pm$5°C is 1.2 million lux·hr using a D65 fluorescent lamp as a light source.
[22] The method according to any one of [13] to [21], wherein the inside of the container is visible.
[23] The method according to any one of [14] to [22], wherein the container is 1) or 2) as follows:

1) a container (preferably, a container whose inside is visible) formed of a polyolefin resin (preferably, polypropylene) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent);
2) a container (preferably, a container whose inside is visible) formed of a polyolefin resin (preferably, polypropylene) having a member (preferably, a heat-shrinkable film (a shrink film)) kneaded with a substance which interferes with transmission of ultraviolet light (more preferably one or more substances selected from the group consisting of an ultraviolet scattering agent and an ultraviolet absorbing agent; particularly preferably one or more substances selected from the group consisting of zinc oxide, titanium oxide and a benzotriazole-based ultraviolet absorbing agent), the member being wound around on a side surface of the container.

[24] A method for suppressing content reduction (for example, adsorption) of a compound represented by the formula (1) or a salt thereof or a solvate thereof in a pharmaceutical preparation comprising an aqueous composition comprising the compound represented by the formula (1) or the salt thereof or the solvate thereof, the aqueous composition being housed in a container formed of a polyolefin resin, comprising the step of:
adding one or more components selected from the group consisting of an acid, a quaternary ammonium type surfactant, and a polyhydric alcohol to the aqueous composition.
[25] The method according to [24], wherein the compound represented by the formula (1) is a compound represented by the formula (2), (5), (5') or (8).
[26] The method according to [24], wherein the compound represented by the formula (1) is a compound represented by the formula (3) or (6).
[27] The method according to [24], wherein the compound represented by the formula (1) is a compound represented by the formula (4) or (7).
[28] The method according to any one of [24] to [27], wherein the polyolefin resin is polyethylene or polypropylene.
[29] The method according to any one of [24] to [28], wherein the acid is one or more selected from the group consisting of boric acid and a salt thereof.
[30] The method according to any one of [24] to [29], wherein the quaternary ammonium type surfactant is benzalkonium chloride or benzododecinium bromide.
[31] The method according to any one of [24] to [30], wherein the polyhydric alcohol is glycerin, sorbitol, propylene glycol, mannitol, or macrogol.

## Examples

Next, the present invention is further described by way of Examples, but the present invention is not limited to these Examples.
In the following Test Examples, the measurements of netarsudil using HPLC were performed using an ODS column as the column, 0.01 mol/L phosphate buffer solution and acetonitrile as the mobile phase, and a UV absorptiometer as the detector (wavelength: 254 nm), respectively.

### [Test Example 1] Light Stability Test Part 1

The light stability of netarsudil blended in an aqueous composition was evaluated by examining the presence or absence of the content reduction of netarsudil due to exposure to light.
In other words, an aqueous composition having the formulation shown in Table 1 was prepared, and the prepared composition was housed in a transparent eye drop container formed of polypropylene (PP) or glass to obtain pharmaceutical preparations of Example or Comparative Example, respectively. Then, each of the pharmaceutical preparations was irradiated using a light stability test device (LT-120A: Nagano Science Co., Ltd.) with a light of 4000

lux under 25°C using a D65 fluorescent lamp as a light source for 75 hours so that cumulative irradiation dose became 300,000 lux·hr.

To examine the presence or absence of the content reduction of netarsudil due to exposure to light, the concentrations of netarsudil in the aqueous composition before and after the exposure to light in the pharmaceutical preparations were measured. The concentration of netarsudil in the aqueous composition was calculated by measuring the ratio of the peak area of the aqueous composition relative to the peak area of a netarsudil solution of a known concentration, using HPLC.

Then, based on the calculated concentration of netarsudil in the aqueous composition, the residual rate (%) of netarsudil was assessed using the following expression:

$$\text{Residual rate (\%)} = \{(\text{Concentration of netarsudil in the aqueous composition after exposure to light})/(\text{Concentration of netarsudil in the aqueous composition before exposure to light})\} \times 100$$

<Container formed of polypropylene (PP)>

The eye drop container formed of polypropylene was used. As to the container, the average value of the transmittance of the light was 68.4% on average in 320 to 380 nm (67.1% on average in 300 to 380 nm; 63.8% on average in 270 to 380 nm; 69.2% on average in 320 to 395 nm; 68.0% on average in 300 to 395 nm; and 64.8% on average in 270 to 395 nm).

The container appearance was nearly transparent and the content inside was visible (for example, the transmittance of the light in the visible light region (450 to 750 nm) was more than 75 % in all wavelengths and 81.9% on average).

The transmittance of the light was measured at 5 nm intervals using a spectrophotometer (U-3900: Hitachi High-Technologies Corporation) after the container was cut into a plate-like piece and set in an optical path so that light enters the piece nearly vertically. The average value of the transmittance of the light was calculated as an average value of the measured transmittance of the light in a predetermined wavelength range at 5 nm intervals.

<Container formed of glass>

The container formed of glass was used. The average value of the transmittance of the light of the container was 71.0% on average in 320 to 380 nm (70.5% on average in 300 to 380 nm; 69.7% on average in 270 to 380 nm; 71.3% on average in 320 to 395 nm; 70.8% on average in 300 to 395 nm; and 70.0% on average in 270 to 395 nm).

The container appearance was nearly transparent and the content inside was visible (for example, the transmittance of the light in the visible light region (450 to 750 nm) was more than 70 % in all wavelengths and 75.4% on average).

The transmittance of the light was measured in the same method as for the container made of polypropylene.

The results are shown in Table 2.

[Table 1]

| Component | Amount (per 100 mL) |
|---|---|
| Netarsudil dimesylate | 0.02848 g (0.02 g in terms of a free form) |
| Boric acid | 0.05 g |
| D-mannitol | 4.7 g |
| Benzalkonium chloride | 0.015 g |
| Sodium hydroxide | q.s. (pH 5.0) |
| Purified water | Balance |

[Table 2]

| | Classification of pharmaceutical preparation and material of container | Cumulative irradiation dose: 300,000 lux·hr |
|---|---|---|
| Residual rate (%) | Example: PP Average value of transmittance of light of 320 to 380 nm: 68.4% | 71.1 |
| | Comparative Example: glass Average value of transmittance of light of 320 to 380 nm: 71.0% | 67.0 |

As shown in the results indicated in Table 2, it was revealed that the residual rate of netarsudil in the aqueous composition decreased due to exposure to light, leading to the content reduction of netarsudil, but when the aqueous composition comprising netarsudil was housed in the container formed of polypropylene (PP), the content reduction was suppressed as compared with the case where the composition was housed in the container formed of glass.

Here, a D65 fluorescent lamp which irradiates a light having a wavelength of 300 nm or more was used as the light source.

As shown in Test Example 2 described below, it is ascertained that the content reduction of netarsudil was significant with the irradiation of a light having a wavelength of shorter than 380 nm, particularly in 320 to 380 nm, suggesting that blocking of the light of these wavelengths is important to ensure the light stability. However, the average value of the transmittance of a light of 320 to 380 nm of the container formed of polypropylene used for the pharmaceutical preparation in Example 1 was 68.4%, which was comparable to the average value of the transmittance of a light of the container formed of glass (71.0%) used in the pharmaceutical preparation in Comparative Example 1, indicating that these containers can block the light of 320 to 380 nm just at the similar level.

This suggests that the suppression of content reduction of netarsudil by the container formed of polypropylene is possibly caused by a different action/mechanism from simply blocking a light having a wavelength which easily reduces the content of netarsudil.

As stated above, it was surprisingly revealed that, by housing the aqueous composition comprising the compound represented by the formula (1) exemplified by netarsudil or a salt thereof or a solvate thereof in a container formed of a polyolefin resin exemplified by polypropylene, despite the container formed of a polyolefin resin described above, the content reduction of the compound due to exposure to light becomes suppressed and the pharmaceutical preparation having an improved light stability can be obtained.

[Test Example 2] Light Stability Test Part 2

To identify the wavelengths of the light causing the content reduction of netarsudil due to exposure to light in the aqueous composition which was observed in Test Example 1, the light having the wavelengths of a certain range were irradiated, and the presence or absence of the content reduction due to exposure to light was evaluated.

In other words, the aqueous composition having the formulation shown in Table 1 was prepared, and the prepared composition was housed in a transparent container formed of glass to obtain a pharmaceutical preparation. Then, using spectral radiation device having an optical filter (spectroscopy unit: HSU-100S, Light Source: MAX-302FBD, both from Asahi Spectra Co., Ltd.), the lights having a wavelength of approximately 270 to 335 nm, 320 to 395 nm, 380 to 410 nm, 430 to 475 nm, 470 to 535 nm or 350 to 800 nm each were irradiated on the pharmaceutical preparation under 25°C. The radiation energy of light at every wavelength was set to approximately 200 W·h/$m^2$.

To examine the presence or absence of the content reduction of netarsudil due to exposure to light, the residual rate (%) of netarsudil was assessed in the same method as in Test Example 1.

The results are shown in Table 3.

[Table 3]

| | Irradiation wavelength (nm) | | | | | |
|---|---|---|---|---|---|---|
| | 270-335 | 320-395 | 380-410 | 430-475 | 470-535 | 350-800 |
| Residual rate (%) | 83.7 | 77.8 | 95.1 | 96.6 | 96.1 | 98.5 |

As shown in the results in Table 3, the content reduction was scarcely observed by irradiation of a light having a wavelength of 380 nm or more, while significant content reduction was observed by irradiation of lights having wavelengths shorter than 380 nm, particularly 320 to 380 nm.

One of the tests to confirm the storage stability of pharmaceutical products is a light stability test, and the guideline of this test defines that D65 light source should be used as the light source. D65 light source is the internationally recognized standard for outdoor daylight as defined in ISO 10977 (1993), and prescribed with the value of the spectral distribution of wavelength of 300 to 830 nm. From these, it is believed that, as to the stability in the typically expected storage condition for pharmaceutical products, it is sufficient if the light having a wavelength of 300 nm or more is considered.

From the above, it was concluded that, for ensuring the light stability of aqueous composition comprising the compound represented by the formula (1) exemplified by netarsudil or a salt thereof or a solvate thereof, it is important to block the light, especially, of wavelength of 320 to 380 nm.

[Test Example 3] Light Stability Test Part 3

Based on the results obtained in Test Example 2, in an attempt to obtain further stabilization, the aqueous composition comprising netarsudil was housed in a container blended with an ultraviolet absorbing agent.

In other words, the aqueous composition having formulation shown in Table 1 was prepared, and the obtained aqueous composition was housed in an eye drop container formed of polypropylene blended with an ultraviolet absorbing agent described below to obtain a pharmaceutical preparation. Then, the obtained pharmaceutical preparation was irradiated using a light stability test equipment (LT-120A: Nagano Science Co., Ltd.) with the light of 4000 lux under 25°C using a D65 fluorescent lamp as a light source for 300 hours so that cumulative irradiation dose becomes 1.2 million lux·hr.

To examine the presence or absence of the content reduction of netarsudil due to exposure to light, the residual rate (%) of netarsudil was assessed in the same method as in Test Example 1.

<Container Formed of Polypropylene Blended with Ultraviolet Absorbing Agent>

The eye drop container formed of polypropylene blended with benzotriazole-based ultraviolet absorbing agent was used. The average value of the transmittance of the light of the container was 18.6% in 320 to 380 nm (18.2% on average in 300 to 380 nm; 21.5% on average in 270 to 380 nm; 23.7% on average in 320 to 395 nm; 22.3% on average in 300 to 395 nm; and 24.3% on average in 270 to 395 nm).

The container appearance was nearly transparent and the content inside was visible (for example, the transmittance of the light in the visible light region (450 to 750 nm) was more than 75 % in all wavelengths and 81.8% on average).

The transmittance of the light was measured at 5 nm intervals using a spectrophotometer (U-3900: Hitachi High-Technologies Corporation) after the container was cut into a plate-like piece and set in an optical path so that light enters the piece nearly vertically. The average value of the transmittance of the light was calculated as an average value of the measured transmittance of the light in a predetermined wavelength range at 5 nm intervals.

In addition, a graph showing the relationship as to this container between the wavelength and transmittance (%) of the light in the range of 270 to 800 nm was shown in Fig. 1.

The results are shown in Table 4.

[Table 4]

| | Properties of container | Cumulative irradiation dose: 1.2 million lux·hr |
|---|---|---|
| Residual rate (%) | Container formed of polypropylene blended with ultraviolet absorbing agent Average value of transmittance of light of 320 to 380 nm: 18.6% | 96.0 |

As shown in the results indicated in Table 4, it was revealed that, by housing the aqueous composition comprising netarsudil in a container formed of polypropylene of which the average value of the transmittance of the light of 320 to 380 nm is 18.6%, the content reduction of netarsudil in the aqueous composition is further significantly suppressed and the light stability is further improved.

[Test Example 4] Light Stability Test Part 4

In the same way as in Test Example 3, in an attempt to obtain further stabilization, the aqueous composition comprising netarsudil was housed in a container formed of polypropylene blended with an ultraviolet scattering agent.

In other words, the test was performed in the same method as in Test Example 3 except that a container formed of polypropylene having a shrink film blended with an ultraviolet scattering agent described below was used instead of the container formed of polypropylene blended with an ultraviolet absorbing agent.

<Container Formed of Polypropylene Having Shrink Film Blended with Ultraviolet Scattering Agent>

An eye drop container formed of polypropylene having a white color shrink film (heat-shrinkable film) (IWATA LABEL Co., Ltd.) kneaded with titanium oxide as an ultraviolet scattering agent in an amount of 40 to 45 % by mass wound around according to an ordinary method on a side surface of the container was used as a container. The average value of transmittance of the light of the shrink film comprising titanium oxide was 0% in 320 to 380 nm (0% on average in any of 300 to 380 nm, 270 to 380 nm, 320 to 395 nm, 300 to 395 nm, and 270 to 395 nm).

Since the shrink film is not transparent, the inside of the container was not visible from the side surface in the original state (e.g., the transmittance of the light was 2.2% on average in the visible light region (450 to 750 nm)). Therefore, slits with the width of about 5 mm were provided on the side surface of the bottom half of the container to allow the inside of the container to be visible (the part provided with the slits was in a state that could not block the light). Further, the shrink film was not wound on bottom surface so that the inside of the container was visible from the bottom surface.

The transmittance of the light was measured at 5 nm intervals using a spectrophotometer (U-3900: Hitachi High-Technologies Corporation) after the film was set in an optical path so that light enters the piece nearly vertically. The average value of the transmittance of the light was calculated as an average value of the measured transmittance of the light in a predetermined wavelength range at 5 nm intervals.

In addition, a graph showing the relationship in this container between the wavelength and transmittance (%) of the light of the range of 270 to 800 nm was shown in Fig. 2.

The results are shown in Table 5.

[Table 5]

| | Properties of container | Cumulative irradiation dose: 1.2 million lux·hr |
|---|---|---|
| Residual rate (%) | Container formed of polypropylene having a wound shrink film comprising titanium oxide Average value of transmittance of light of 320 to 380 nm: 0% | 100 |

As shown in the result listed in Table 5, it was revealed that, by housing the aqueous composition comprising netarsudil in a container formed of polypropylene of which the average value of the transmittance of the light having a wavelength of 320 to 380 nm is 0%, the content reduction of netarsudil in the aqueous composition is almost completely suppressed and the light stability is further improved.

As shown in the results listed Test Examples 3 and 4, it was confirmed that, by housing the aqueous composition comprising the compound represented by the formula (1) exemplified by netarsudil or a salt thereof or a solvate thereof in a container formed of a polyolefin resin which blocks a light having a wavelength of 320 to 380 nm, the light stability of the compound represented by the formula (1) in the aqueous composition is furthermore improved.

[Test Example 5] Heat Stability Test

The heat stability of netarsudil in the aqueous composition was assessed by examining the presence or absence of content reduction of netarsudil after stored under high temperature for a certain period.

In other words, the aqueous composition having the formulation shown in Table 6 was prepared with an ordinary method, and then the obtained composition was housed in a container formed of low-density polyethylene (LDPE), high density polyethylene (HDPE) or polypropylene (PP) to obtain pharmaceutical preparations of Examples 1 to 3, respectively. In addition, the same aqueous composition was housed in a container formed of glass to prepare a pharmaceutical preparation of Comparative Example.

Each of the obtained pharmaceutical preparations was stored at 80°C for a week. To examine the presence or absence of content reduction of netarsudil after storage, the concentrations of netarsudil in the aqueous composition before and after storage were calculated for each of the pharmaceutical preparations by the same method as in Test Example 1.

Then, based on the calculated concentration of netarsudil in the aqueous composition, the residual rate (%) of netarsudil was assessed using the following expression:

Residual rate (%) = {(Concentration of netarsudil in the aqueous composition after storage)/(Concentration of netarsudil in the aqueous composition before storage)} × 100

The results are shown in Table 7.

[Table 6]

| Component | Amount (per 100 mL) |
|---|---|
| Netarsudil dimesylate | 0.02848 g (0.02 g in terms of a free form) |
| Boric acid | 0.05 g |
| Sodium hydroxide | q.s. (pH 5.0) |
| Purified water | Balance |

[Table 7]

| | Class of pharmaceutical preparation and material of container | After storage at 80°C for a week |
|---|---|---|
| Residual rate (%) | Example 1: LDPE | 72.1 |
| | Example 2: HDPE | 71.4 |
| | Example 3: PP | 70.7 |
| | Comparative Example: glass | 47.5 |

As shown in the results listed in Table 7, when the aqueous composition comprising netarsudil was housed in the container formed of polyethylene (PE) (Examples 1, 2) or the container formed of polypropylene (PP)(Example 3), the content reduction due to storage under high temperature was suppressed relative to the case housed in a container formed of glass (Comparative Example 1).

From the results of Test Example 5, it was revealed that when the aqueous composition comprising the compound represented by the formula (1) exemplified by netarsudil or a salt thereof or a solvate thereof is housed in a container formed of a polyolefin resin, the content reduction due to storage under high temperature is relatively suppressed, and a pharmaceutical preparation having excellent storage stability is obtained.

[Test Example 6] Adsorption Suppression Test Part 1

To investigate the presence or absence of adsorption of netarsudil to the container formed of a polyolefin resin and the means for suppressing the adsorption, a piece formed of a polyolefin resin was placed in an aqueous composition comprising netarsudil and stored for a certain period of time and then the presence or absence of the content reduction of netarsudil in the aqueous composition was examined.

In other words, a variety of aqueous compositions shown in Table 8 were prepared with an ordinary method, and 5 mL of the obtained composition each was housed in a glass container, and then, into the composition, three resin pieces (each having a size of 1 cm x 2 cm) formed of polypropylene (PP) were immersed to obtain a pharmaceutical preparation of Reference Example 1 or 2. In addition, a pharmaceutical preparation was prepared in the same way as Reference Example 2 except for not immersing resin pieces formed of polypropylene to obtain a pharmaceutical preparation of Reference Example 3.

Each of the obtained pharmaceutical preparations was stored at 60°C for a week. To examine the presence or absence of content reduction of netarsudil after storage, the concentrations of netarsudil in the aqueous composition before and after storage were calculated in each pharmaceutical preparation, by the same method as in Test Example 1.

Then, based on the calculated concentration of netarsudil in the aqueous composition, the residual rate (%) of netarsudil was assessed using the same expression as in Test Example 5.

The results are shown in Table 8.

[Table 8]

| | | Reference Example 1 | Reference Example 2 | Reference Example 3 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Boric acid | 0.05 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 84.5 | 74.9 | 83.1 |

As shown in the results listed in Table 8, from the comparison between Reference Examples 2 and 3, by immersing the resin piece formed of polypropylene, the content of netarsudil in the aqueous composition after storage under high temperature was further reduced. Since such content reduction was attributed to the immersion of the resin piece formed of polypropylene, it was presumed that such reduction is caused by the adsorption of netarsudil to the resin piece formed of polypropylene.

However, from the comparison between Reference Examples 1 and 2, it was revealed that, by adding boric acid to the aqueous composition comprising netarsudil, the residual rate of netarsudil becomes a similar level to Reference Example 3, showing that the content reduction is suppressed.

From the test results above, it was revealed that, by adding the acids exemplified by boric acid, to an aqueous composition comprising the compound represented by the formula (1) exemplified by netarsudil or a salt thereof or a solvate thereof, the content reduction (adsorption), which may occur when the aqueous composition is housed in a container formed of a polyolefin resin exemplified by polypropylene and stored under high temperature, is relatively suppressed.

[Test Example 7] Adsorption Suppression Test Part 2

This test was performed in the same method as in Test Example 6 except for changing the formulation of the aqueous composition to the formulation shown in Table 9.

The results are shown in Table 9.

[Table 9]

| | | Reference Example 4 | Reference Example 5 | Reference Example 6 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | Benzalkonium chloride | 0.015 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 85.4 | 74.9 | 83.1 |

As shown in the results listed in Table 9, from the comparison between Reference Examples 4 and 5, by adding benzalkonium chloride to the aqueous composition comprising netarsudil, as in the case of adding boric acid, the residual rate of netarsudil becomes a similar level to Reference Example 6, showing that the content reduction is suppressed.

From the test results above, it was revealed that, by adding the quaternary ammonium type surfactant exemplified by benzalkonium chloride to the aqueous composition comprising the compound represented by the formula (1) exemplified by netarsudil or a salt thereof or a solvate thereof, the content reduction (adsorption), which may occur when the aqueous composition housed in a container formed of a polyolefin resin exemplified by polypropylene is stored under high temperature, is relatively suppressed.

[Test Example 8] Adsorption Suppression Test Part 3

This test was performed in the same method as in Test Example 6 except for changing the formulation of the aqueous composition to the formulation shown in Table 10.
The results are shown in Table 10.

[Table 10]

| | | Reference Example 7 | Reference Example 8 | Reference Example 9 |
|---|---|---|---|---|
| Aqueous composition (amount: per 100 mL) | Netarsudil dimesylate | 0.02848 g (0.02 g in terms of a free form) | 0.02848 g (0.02 g in terms of a free form) | 0.02848 g (0.02 g in terms of a free form) |
| | D-mannitol | 4.7 g | - | - |
| | Sodium hydroxide | q.s. (pH 5.0) | q.s. (pH 5.0) | q.s. (pH 5.0) |
| | Purified water | Balance | Balance | Balance |
| Presence or absence of immersion of resin piece formed of PP | | Presence | Presence | Absence |
| Residual rate (%) | | 88.1 | 74.9 | 83.1 |

As shown in the results indicated in Table 10, from the comparison between Reference Examples 7 and 8, by adding D-mannitol to the aqueous composition comprising netarsudil, as in the case of adding boric acid, the residual rate of netarsudil becomes a similar level to Reference Example 9, showing that the content reduction is suppressed.
From the test results above, it was revealed that, by adding the polyhydric alcohol exemplified by D-mannitol to the aqueous composition comprising the compound represented by the formula (1) exemplified by netarsudil or a salt thereof or a solvate thereof, the content reduction (presumptively, adsorption), which may occur when the aqueous composition housed in a container formed of a polyolefin resin exemplified by polypropylene is stored under high temperature, is relatively suppressed.

[Preparation Examples 1 to 36]

Pharmaceutical preparations of Preparation Examples 1 to 36 can be prepared by preparing aqueous compositions each comprising the components in amounts ((g) per 100 mL of the aqueous composition) shown in Tables 11 to 19 with an ordinary method, and housing the prepared aqueous composition each in an eye drop container formed of high-density polyethylene.

[Table 11]

| | Preparation Examples | | | |
|---|---|---|---|---|
| | 1 | 2 | 3 | 4 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |

(continued)

| | Preparation Examples | | | |
|---|---|---|---|---|
| | 1 | 2 | 3 | 4 |
| Citric acid | | | | |
| Sodium citrate | | | | |
| Acetic acid | | | | |
| Sodium acetate | | | | |
| Sodium edetate | | 0.01 | 0.01 | |
| D-mannitol | 4.7 | | | |
| Glycerin | | 2.5 | | |
| Propylene glycol | | | 1.7 | |
| Xylitol | | | 0.7 | |
| Macrogol 6000 | | | | 1 |
| Macrogol 4000 | | | | 4 |
| Sorbitol | | | | |
| Trometamol | | | | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 12]

| | Preparation Examples | | | |
|---|---|---|---|---|
| | 5 | 6 | 7 | 8 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Boric acid | | | | |
| Borax | | | | |
| Sodium dihydrogen phosphate | | | | |
| Disodium hydrogen phosphate | | | | |

(continued)

| | Preparation Examples | | | |
|---|---|---|---|---|
| | 5 | 6 | 7 | 8 |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 6000 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 13]

| | Preparation Examples | | | |
|---|---|---|---|---|
| | 9 | 10 | 11 | 12 |
| Netarsudil dimesylate | 0.01424 g (0.01 g in terms of a free form | 0.05696 g (0.04 g in terms of a free form) | 0.02848 g (0.02 g in terms of a free form) | 0.02848 g (0.02 g in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |

(continued)

| | Preparation Examples | | | |
|---|---|---|---|---|
| | 9 | 10 | 11 | 12 |
| Citric acid | | | | |
| Sodium citrate | | | | |
| Acetic acid | | | | |
| Sodium acetate | | | | |
| Sodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 6000 | 1 | | | |
| Macrogol 4000 | | 2 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 14]

| | Preparation Examples | | | |
|---|---|---|---|---|
| | 13 | 14 | 15 | 16 |
| Netarsudil dimesylate | 0.02848 g (0.02 g in terms of a free form) | 0.02848 g (0.02 g in terms of a free form) | 0.01424 g (0.01 g in terms of a free form) | 0.05696 g (0.04 g in terms of a free form) |
| Boric acid | | | | |
| Borax | | | | |
| Sodium dihydrogen phosphate | | | | |
| Disodium hydrogen phosphate | | | | |

(continued)

| | Preparation Examples | | | |
|---|---|---|---|---|
| | 13 | 14 | 15 | 16 |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 6000 | | 1 | | |
| Macrogol 4000 | | 2 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 15]

| | Preparation Examples | | | |
|---|---|---|---|---|
| | 17 | 18 | 19 | 20 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |

(continued)

| | Preparation Examples | | | |
|---|---|---|---|---|
| | 17 | 18 | 19 | 20 |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | 2.5 | 1 | | 4 |
| Macrogol 4000 | | | | 1 |
| Sorbitol | | | | |
| Trometamol | | | | |
| Benzalkonium chloride | 0.2 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.0 | 5.0 | 5.0 |

[Table 16]

| | Preparation Examples | | | |
|---|---|---|---|---|
| | 21 | 22 | 23 | 24 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 400 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |

(continued)

| | Preparation Examples | | | |
|---|---|---|---|---|
| | 21 | 22 | 23 | 24 |
| Purified water | Total Amount 100 mL | Total Amount 100 mL | Total Amount 100 mL | Total Amount 100 mL |
| pH | 6.0 | 5.5 | 6.0 | 5.5 |

[Table 17]

| | Preparation Examples | | | |
|---|---|---|---|---|
| | 25 | 26 | 27 | 28 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 400 | | 2 | | |
| Macrogol 4000 | | 1 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |

(continued)

| | Preparation Examples | | | |
|---|---|---|---|---|
| | 25 | 26 | 27 | 28 |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 7.0 | 6.0 | 7.0 | 6.0 |

[Table 18]

| | Preparation Examples | | | |
|---|---|---|---|---|
| | 29 | 30 | 31 | 32 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 400 | | 2 | | |
| Macrogol 4000 | | 1 | | |
| Sorbitol | | | | 0.5 |

(continued)

| | Preparation Examples | | | |
|---|---|---|---|---|
| | 29 | 30 | 31 | 32 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 19]

| | Preparation Examples | | | |
|---|---|---|---|---|
| | 33 | 34 | 35 | 36 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Timolol maleate | 6.83 mg (5.0 mg in terms of a free form) | 6.83 mg (5.0 mg in terms of a free form) | 6.83 mg (5.0 mg in terms of a free form) | 6.83 mg (5.0 mg in terms of a free form) |
| Boric acid | 0.04 | 0.05 | | |
| Borax | 0.01 | | | |
| Sodium dihydrogen phosphate | | 0.031 | | |
| Disodium hydrogen phosphate | | 0.07 | | |
| Citric acid | | | 0.05 | |
| Sodium citrate | | | 0.03 | |
| Acetic acid | | | | 0.001 |
| Sodium acetate | | | | 0.2 |
| Sodium edetate | 0.01 | 0.01 | 0.01 | 0.01 |
| D-mannitol | 4.7 | | | |
| Glycerin | | 2.5 | | |
| Propylene glycol | | | 1.7 | |

(continued)

| | Preparation Examples | | | |
|---|---|---|---|---|
| | 33 | 34 | 35 | 36 |
| Xylitol | | | 0.7 | |
| Macrogol 400 | | 1 | | |
| Macrogol 4000 | | | 1 | |
| Sorbitol | | | | 3 |
| Trometamol | | | | 15 |
| Benzalkonium chloride | 0.015 | 0.03 | 0.05 | |
| Benzododecinium bromide | | | | 0.01 |
| Benzethonium chloride | | | | 0.01 |
| Methyl cellulose | 0.5 | 2 | | |
| Gellan gum | | | 0.2 | 0.6 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 6.0 | 7.0 | 6.0 |

[Preparation Examples 37 to 72]

Pharmaceutical preparations of Preparation Examples 37 to 72 can be prepared in the same way as in Preparation Examples 1 to 36 except for using an eye drop container formed of polypropylene instead of that formed of high-density polyethylene.

[Preparation Examples 73 to 108]

Pharmaceutical preparations of Preparation Examples 73 to 108 can be prepared in the same way as in Preparation Examples 1 to 36 except for using an eye drop container formed of cyclic polyolefin instead of that formed of high-density polyethylene.

[Preparation Examples 109 to 144]

Pharmaceutical preparations of Preparation Examples 109 to 144 can be prepared in the same way as in Preparation Examples 1 to 36 except for using an eye drop container formed of low-density polyethylene instead of that formed of high-density polyethylene.

[Preparation Examples 145 to 288]

Pharmaceutical preparations of Preparation Examples 145 to 288 can be prepared with an ordinary method by using 0.5 g of verosudil monohydrochloride in a free form instead of netarsudil dimesylate in the Preparation Examples 1 to 144.

[Preparation Examples 289 to 432]

Pharmaceutical preparations of Preparation Examples 289 to 432 can be prepared with an ordinary method by using 0.7 g of verosudil monohydrochloride in a free form instead of netarsudil dimesylate in the Preparation Examples

1 to 144.

[Preparation Examples 433 to 576]

Pharmaceutical preparations of Preparation Examples 433 to 576 can be prepared with an ordinary method by using 0.02 g of a compound represented by the formula (8) instead of netarsudil dimesylate in the Preparation Examples 1 to 144.

Industrial Applicability

According to the present invention, a pharmaceutical preparation having excellent stability can be provided, and it can be suitably used in the pharmaceutical industry and the like.

## Claims

**1.** A pharmaceutical preparation comprising:
an aqueous composition comprising a compound represented by the following formula (1)

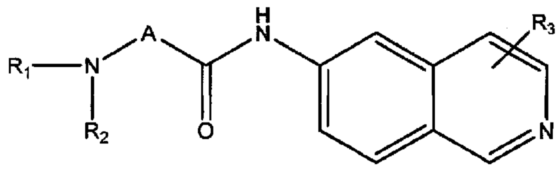

(1)

(in the formula, $R_1$ and $R_2$ each independently represent a hydrogen atom or a $C_1$-$C_4$ alkyl group,

$R_3$ represents a hydrogen atom or a hydroxy group, and
"A" represents -$CH(R_4)$- or -$CH_2$-$CH(R_4)$- (wherein $R_4$ represents an optionally substituted $C_6$-$C_{10}$ aryl group, or an optionally substituted 5 to 10-membered heteroaryl group), and
the formula (1) also includes a tautomer thereof) or a salt thereof or a solvate thereof, the aqueous composition being housed in a container formed of a polyolefin resin.

**2.** The pharmaceutical preparation according to claim 1, wherein the compound represented by the formula (1) is a compound represented by the following formula (2), (5), (5') or (8) :

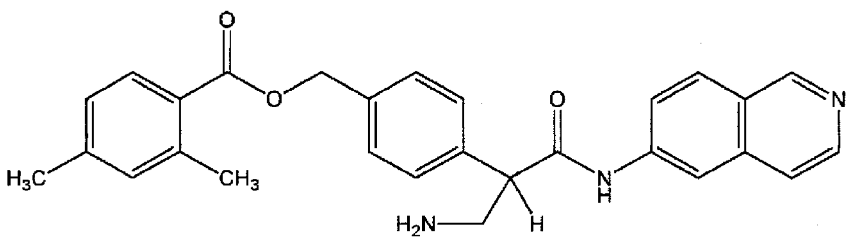

(2) ;

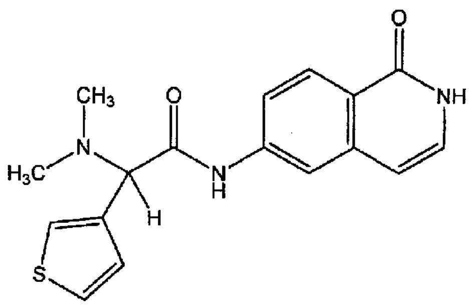

(5) ;

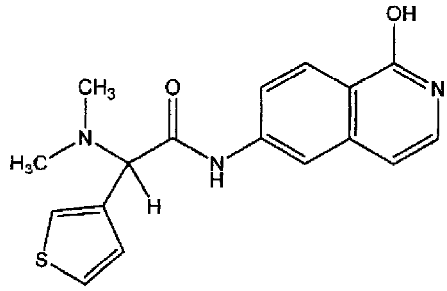

(5') ;

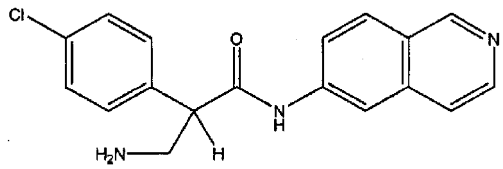

(8)

3. The pharmaceutical preparation according to claim 1, wherein the compound represented by the formula (1) is a compound represented by the following formula (3):

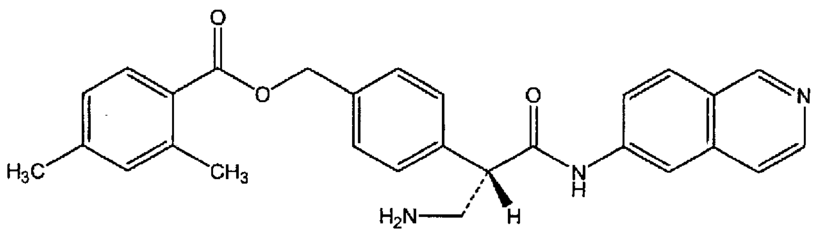

(3) .

4. The pharmaceutical preparation according to any one of claims 1 to 3, wherein the polyolefin resin is polyethylene or polypropylene.

5. The pharmaceutical preparation according to any one of claims 1 to 4, wherein the container is a container which blocks a light having a wavelength of from 320 to 380 nm.

6. The pharmaceutical preparation according to claim 5, wherein a residual rate of the compound represented by the formula (1) in the aqueous composition after irradiation is 80% or more, when the aqueous composition is irradiated with a light so that a cumulative irradiation dose at a temperature of 25±5°C is 1.2 million lux·hr using a D65 fluorescent lamp as a light source.

7. The pharmaceutical preparation according to claim 5 or 6, wherein the container is a container comprising a substance which interferes with transmission of ultraviolet light.

8. The pharmaceutical preparation according to any one of claims 5 to 7, wherein the container is a container having a member comprising a substance which interferes with transmission of ultraviolet light.

9. A method for improving light stability of a compound represented by the following formula (1):

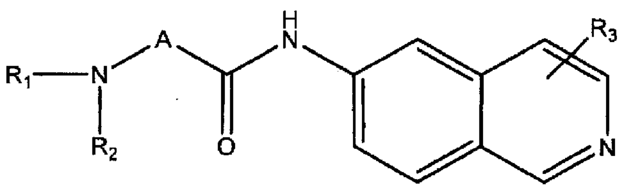

(1)

(in the formula, $R_1$ and $R_2$ each independently represent a hydrogen atom or a $C_1$-$C_4$ alkyl group,

$R_3$ represents a hydrogen atom or a hydroxy group, and
"A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof) or a salt thereof or a solvate thereof in an aqueous composition, comprising a step of:
housing the aqueous composition comprising the compound represented by the formula (1) or a salt thereof or a solvate thereof in a container formed of a polyolefin resin.

**10.** The method according to claim 9, wherein the compound represented by the formula (1) is a compound represented by the formula (2), (5), (5') or (8):

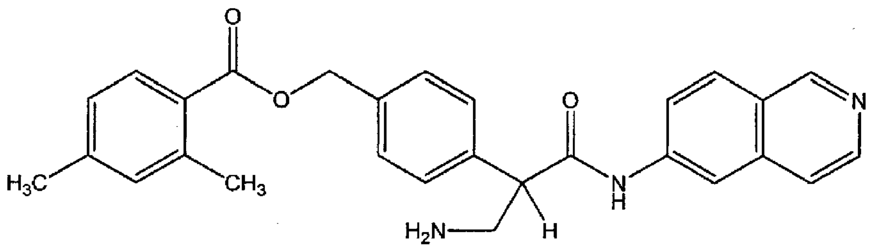

(2) ;

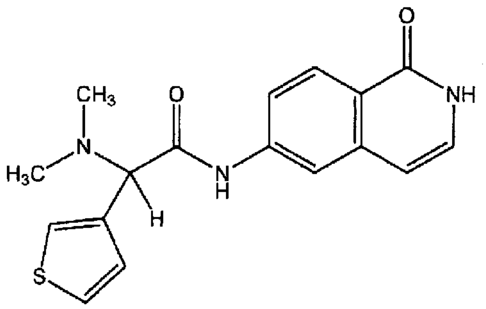

(5) ;

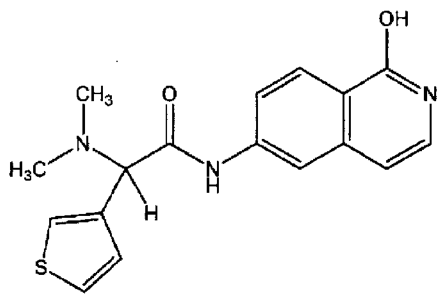

(5') ;

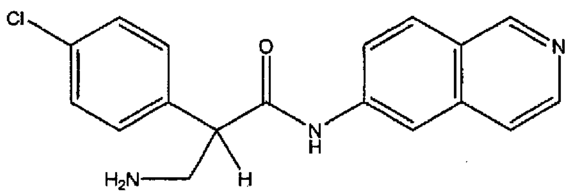

(8) .

**11.** The method according to claim 9, wherein the compound represented by the formula (1) is a compound represented by the following formula (3):

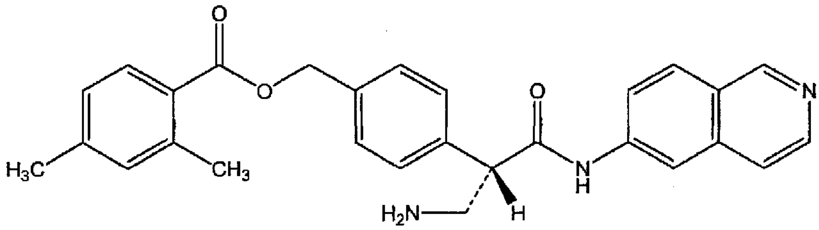

(3) .

12. The method according to any one of claims 9 to 11, wherein the polyolefin resin is polyethylene or polypropylene.

13. The method according to any one of claims 9 to 12, wherein the container is a container which blocks a light having a wavelength of from 320 to 380 nm.

14. The method according to claim 13, wherein a residual rate of the compound represented by the formula (1) in the aqueous composition after irradiation is 80% or more, when the aqueous composition is irradiated with a light so that a cumulative irradiation dose at a temperature of 25±5°C is 1.2 million lux·hr using a D65 fluorescent lamp as a light source.

15. The method according to claim 13 or 14, wherein the container is a container comprising a substance which interferes with transmission of ultraviolet light.

16. The method according to any one of claims 13 to 15, wherein the container is a container having a member comprising a substance which interferes with transmission of ultraviolet light.

[Figure 1]

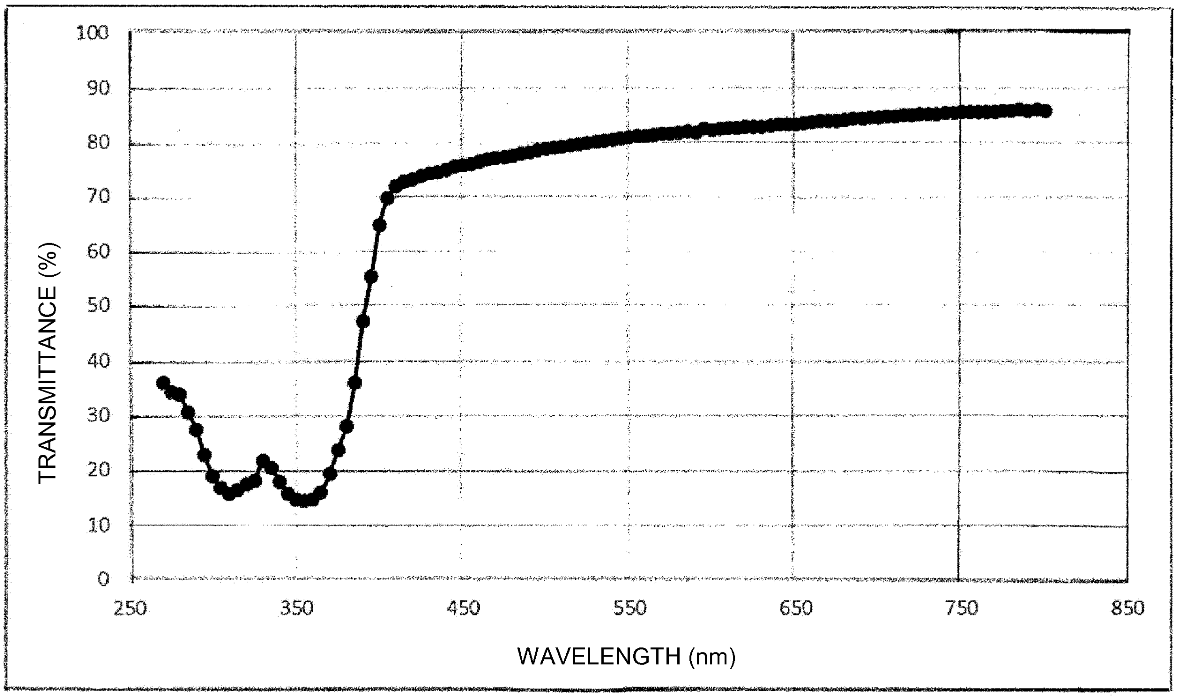
TRANSMITTANCE (%)
100
90
80
70
60
50
40
30
20
10
0
250
350
450
550
650
750
850
WAVELENGTH (nm)

[Figure 2]

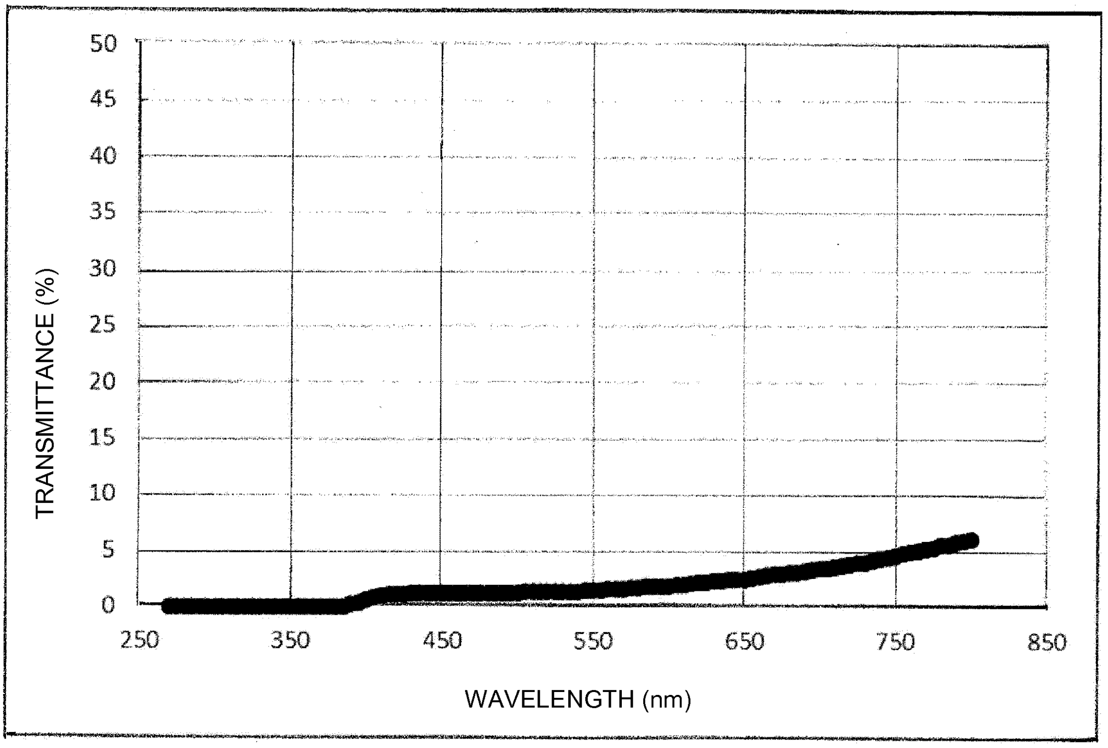

50
45
40
35
30
25
20
15
10
5
0
TRANSMITTANCE (%)
250
350
450
550
650
750
850
WAVELENGTH (nm)

**INTERNATIONAL SEARCH REPORT**

International application No. PCT/JP2018/007582

A. CLASSIFICATION OF SUBJECT MATTER
Int.Cl. A61K31/472(2006.01)i, A61J1/05(2006.01)i, A61K9/08(2006.01)i, A61K31/4725(2006.01)i, A61K47/04(2006.01)i, A61K47/10(2006.01)i, A61P27/02(2006.01)i, A61P27/06(2006.01)i, B65D1/00(2006.01)i, B65D65/20(2006.01)i

According to International Patent Classification (IPC) or to both national classification and IPC

B. FIELDS SEARCHED

Minimum documentation searched (classification system followed by classification symbols)
Int.Cl. A61K31/472, A61J1/05, A61K9/08, A61K31/4725, A61K47/04, A61K47/10, A61P27/02, A61P27/06, B65D1/00, B65D65/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGlSTY/MEDLlNE/EMUASE/BIOSIS (STN), WPI, JSTPlus/JMEDPlus/JST7580 (JDreamIII), Science Direct, PubMed

C. DOCUMENTS CONSIDERED TO BE RELEVANT

| Category* | Citation of document, with indication, where appropriate, of the relevant passages | Relevant to claim No. |
|---|---|---|
| Y | JP 2016-515520 A (AERIE PHARMACEUTICALS, INC.) 30 May 2016, claims, paragraphs [0199], [0200], examples 1-6 & WO 2014/144781 A1, claims, paragraphs [0191], [0192], examples 1-6 & EP 2976080 A1 & US 2014/0275160 A1 | 1-16 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 22 March 2018 (22.03.2018) | 03 April 2018 (03.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/007582

C (Continuation). DOCUMENTS CONSIDERED TO BE RELEVANT

| Category* | Citation of document, with indication, where appropriate, of the relevant passages | Relevant to claim No. |
|---|---|---|
| Y | ELLIS, Eydie Miller et al., "Ocular hypotensive effect of the novel EP3/FP agonist ONO-9054 versus Xalatan: results of a 28-day, double-masked, randomised study", British Journal of Ophthalmology, 20 September 2016, vol. 101, pp. 796-800, Study design, ISSN:1468-2079 | 1-16 |
| A | WO 2016/047720 A1 (KOWA CO., LTD.) 31 March 2016 & US 2017/0266080 A1 & EP 3199162 A1 | 1-16 |
| A | WO 2005/087237 A1 (ASAHI KASEI PHARMA CORPORATION) 22 September 2005 & US 2008/0234483 A1 & EP 1726306 A1 | 1-16 |
| A | JP 2016-138085 A (ROHTO PHARMACEUTICAL CO., LTD.) 04 August 2016 (Family: none) | 1-16 |
| A | LEVY, Brian et al., "Ocular hypotensi ve safety and systemic absorption of AR-13324 ophthalmic solution in normal volunteers", American Journal of Ophthalmology, May 2015, vol. 159, no. 5, pp. 980-985.el, ISSN:0002-9394 | 1-16 |
| A | REN, Ruiyi et al., "Netarsudil increases outflow facility in human eyes through multiple mechanisms", Investigative Ophthalmology and Visual Science, November 2016, vol. 57, no. 14, pp. 6197-6209, ISSN:1552-5783 | 1-16 |
| P, X | RHOPRESSA™ (netarsudil ophthalmic solution) Label, NDA 208254, Reference ID:4194833, December 2017, pp. 4-10 | 1-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012525386 A
- WO 2009091898 A
- JP 2016515520 A


### Non-patent literature cited in the description


- **BACHARACH J. et al.** *Ophthalmology,* 2015, vol. 122 (2), 302-7
- **STURDIVANT JM. et al.** *Bioorg Med Chem Lett.,* 2016, vol. 26 (10), 2475-80
- **WILLIAMS RD. et al.** *Am. J. Ophthalmol.,* 2011, vol. 152 (5), 834-41
- Pharmaceutical Excipients Dictionary. Yakuji Nippo, 2016
- General Notices. Japanese Pharmacopoeia